# EUROPEAN PATENT SPECIFICATION

(11) **EP 1 907 851 B1**
(45) Date of publication and mention of the grant of the patent: **19.10.2011**
(21) Application number: 06796239.9
(22) Date of filing: 28.07.2006
(51) Int. Cl.: G01N 33/564

(54) **STIMULATORY AUTO-ANTIBODIES TO THE PDGF RECEPTOR AS PATHOLOGY MARKER AND THERAPEUTIC TARGET**
STIMULATORISCHE AUTOANTIKÖRPER GEGEN DEN PDGF-REZEPTOR ALS PATHOLOGISCHE MARKER UND THERAPEUTISCHES TARGET
AUTO-ANTICORPS STIMULATEURS DU RECEPTEUR PDGF UTILISES COMME MARQUEUR PATHOLOGIQUE ET COMME CIBLE THERAPEUTIQUE

(30) Priority: 28.07.2005 US 703377 P
(43) Date of publication of application: 09.04.2008
(73) Proprietor: Avvedimento, Vittorio Enrico, 81100 Caserta (IT); Armando, Gabrielli, 60020 Ancona (IT); Santillo, Mariarosa, 80128 Napoli (IT); Funaro, Ada, 10129 Torino (IT); Luchetti, Michele, 60124 Ancona (IT); Svegliati Baroni, Silvia, 60123 Ancona (IT)
(72) Inventor: Avvedimento, Vittorio Enrico, 81100 Caserta (IT); Armando, Gabrielli, 60020 Ancona (IT); Santillo, Mariarosa, 80128 Napoli (IT); Funaro, Ada, 10129 Torino (IT); Luchetti, Michele, 60124 Ancona (IT); Svegliati Baroni, Silvia, 60123 Ancona (IT)
(74) Representative: Capasso, Olga
(86) International application number: PCT/IT2006/000587
(87) International publication number: WO 2007/013124

(56) References cited:
- WO-A-02/33408
- CHIZZOLINI CARLO ET AL: "Autoantibodies to fibroblasts induce a proadhesive and proinflammatory fibroblast phenotype in patients with systemic sclerosis." ARTHRITIS AND RHEUMATISM JUN 2002, vol. 46, no. 6, June 2002 (2002-06), pages 1602-1613, XP002421599 ISSN: 0004-3591
- CEPEDA EDUARDO J ET AL: "Autoantibodies in systemic sclerosis and fibrosing syndromes: clinical indications and relevance" CURRENT OPINION IN RHEUMATOLOGY, vol. 16, no. 6, November 2004 (2004-11), pages 723-732, XP009079600 ISSN: 1040-8711
- HARRIS MICHELLE L ET AL: "Autoimmunity in scleroderma: The origin, pathogenetic role, and clinical significance of autoantibodies." CURRENT OPINION IN RHEUMATOLOGY, vol. 15, no. 6, November 2003 (2003-11), pages 778-784, XP009079576 ISSN: 1040-8711
- BARONI SILVIA SVEGLIATI ET AL: "Stimulatory autoantibodies to the PDGF receptor in systemic sclerosis." THE NEW ENGLAND JOURNAL OF MEDICINE 22 JUN 2006, vol. 354, no. 25, 22 June 2006 (2006-06-22), pages 2667-2676, XP009079568 ISSN: 1533-4406
- OLIVIERI ATTILIO ET AL: "Stimulatory auto-antibodies to the PDGF receptor in patients with chronic GVHD." BLOOD, vol. 106, no. 11, Part 1, November 2005 (2005-11), page 137A, XP009079617 & 47TH ANNUAL MEETING OF THE AMERICAN-SOCIETY-OF-HEMATOLOGY; ATLANTA, GA, USA; DECEMBER 10 -13, 2005 ISSN: 0006-4971

## Description

### TECHNICAL BACKGROUND

Serum of patients affected by an autoimmune disease, systemic sclerosis or sclerodermia, contains stimulating auto-antibodies to the human PDGF receptor (PDGFR). PDGFR is a homo or hetero dimeric molecule of PDGFRA NP008197, (SWProt P16234) and PDGFRB NPOO26000 (SWProt P09619). These antibodies can be used as diagnostic tool for the disease. Moreover treatment with inhibitors of anti-PDGFR could represent a therapeutic approach to the disease.

Presently the disease, although is rare (1/10,000), is lethal and no specific therapy is available. The diagnosis is essentially based on clinical symptoms and no-specific assay is available.

The detection of such auto antibodies can be used for the diagnosis of 2 other diseases, more frequent than systemic sclerosis:
- Raynaud phenomenon, which is present in 20% of the population and generally is benign, though some evolve to systemic sclerosis; the antibody test can discriminate between primary and secondary Raynaud, which is an early sign of systemic sclerosis.
- Graft-Versus-Host-Raction (GVHR). This is a very frequent complication following allogenic organ transplants, mainly bone marrow. We have detected the presence of the antibodies described above in all patients (15) (so far analyzed) with chronic GVH and fibrosis

Systemic sclerosis (scleroderma; SSc) is a disorder characterized by fibrosis of the skin and visceral organs (1). The primary cause of the disease is unknown and at present no definite and specific hypothesis explains all aspects of the illness. Some features of scleroderma phenotype are well established and represent the hallmarks of the disease: 1. endothelial cell damage and 2. excessive extracellular matrix production (2). Cellular scleroderma phenotype is characterized by the oxidative stress and the generation by fibroblasts of large amounts of reactive oxygen species (ROS) (3-6). ROS are key cell transducers of fibroblast proliferation (7) and collagen gene expression (7). The large production of ROS *via* Ha-Ras and ERK 1-2 induced collagen gene transcription and senescence in normal fibroblasts (7).

Platelet-derived growth factor (PDGF) can induce ROS and Ras-ERK1/2 signalling (9); moreover IgG derived from scleroderma patients (SSc IgG) were shown to react with human fibroblasts (10).

### DESCRIPTION OF THE INVENTION

The authors of the invention have searched for Ha/ERK1-2 and ROS stimulatory molecules in the serum of SSc patients.

They provide evidence that stimulatory IgG auto-antibodies directed to the PDGF receptor (PDGFR), by triggering PDGFR activation, induce ROS *via* Ha-Ras and ERK1-2 signalling and are responsible for fibroblast activation and endothelial cell apoptosis, which are the distinctive features of systemic sclerosis.

Therefore they found a novel level of regulation of *Ras* proteins, dependent on ERK1/2 signalling. Specifically, they found that PDGF and ROS induce Ha-*Ras* in primary fibroblasts. This has revealed a novel and hitherto unknown pathway, which links ROS to *Ras* protein levels through ERK1/2. They found a remarkable example of this circuitry *in vivo* in cells derived from patients affected by systemic sclerosis. This signalling pathway is initiated by stimulation of PDGF receptor and maintained by ROS-ERK1/2 signals. Systemic sclerosis cells produce excess of ROS (12) and maintain active *Ras*-ERK1/2. These cells accumulate DNA damage, activate ROS-dependent genes and become prone to stress-induced apoptosis. Inhibition of ROS, *Ras* or ERK1/2 down-regulates the loop and restores the normal phenotype in systemic sclerosis fibroblasts. These data point to *Ras* a key sensor of cellular ROS and suggest a molecular tool for the diagnosis and therapy of systemic sclerosis. Therefore it is an object of the invention an *in vitro* method for detecting the presence in a body sample of auto-antibodies for the PDGF receptor characterised by:
a) incubating the body sample with an effective amount of a specific ligand for such auto-antibodies in conditions allowing the binding and the formation of a complex;
b) detecting the bound auto-antibodies, or the complex, if present.

Preferably the specific ligand is the PDGF receptor or immunoreactive fragments or derivatives thereof.

More preferably said immunoreactive fragments belong to the extracellular region of PDGFrA and/or PDGFrB, **even more preferably** said immunoreactive fragments belonging to the extracellular region of PDGFrA and/or PDGFrB are comprised in the first 550 amino acids from the NH terminus of the amino acid sequence of the human PDGFrA NP008197, (SWProt P16234, SEQ ID No. 7) and/or PDGFrB NPOO26000 (SWProt P09619, SEQ ID No.8).

### SWProt P16234, SEQ ID No. 7

### SWProt P09619, SEQ ID No. 8

The method of the invention is suitable for the diagnosis and prognosis of the systemic sclerosis, and for discriminating between Raynaud phenomenon and systemic sclerosis.

The method of the invention is suitable for the diagnosis and prognosis of the Graft-Versus-Host-Reaction (GVHR).

It is another object of the invention a diagnostic kit for the method according to any of previous claims comprising:
a) the PDGF receptor or immunoreactive fragments thereof for auto-antibodies for the PDGF receptor;
b) detecting means for bound auto-antibodies, or for the complex ligand-autoantibodies.

Preferably the immunoreactive fragments belong to the extracellular region of PDGFrA and/or PDGFrB.

More preferably such immunoreactive fragments belong to the extracellular region of PDGRA and/or PDGFRB, most preferably are comprised in the first 550 amino acids from the NH terminus of the amino acid sequence of the human PDGFRA NP008197, (SWProt P16234, SEQ ID No. 7) and/or PDGFRB NPOO26000 (SWProt P09619, SEQ ID No. 8).

It is another object of the invention the PDGF receptor or immunoreactive fragments thereof for use for treating the Graft-Versus-Host-Reaction (GVHR) in patients with scleroderma-like skin lesions after allogenic bone marrow translaplantation.

It is another object of the invention the PDGF receptor or immunoreactive fragments thereof for use for treating the systemic sclerosis.

Preferably such immunoreactive fragments belong to the extracellular region of PDGRA and/or PDGFRB, most preferably are comprised in the first 550 amino acids from the NH terminus of the amino acid sequence of the human PDGFRA NP008197, (SWProt P16234, SEQ ID No. 7) and/or PDGFRB NPOO26000 (SWProt P09619, SEQ ID No. 8).

It is another object of the invention a pharmaceutical composition comprising an effective amount of PDGF receptor or immunoreactive fragments thereof, and proper diluents, and /or excipients, and/or adjuvants for use in treating the Graft-Versus-Host-Reaction (GVHR) **in patients with scleroderma-like skin lesions after allogenic bone marrow translaplantation** or the systemic sclerosis. Preferably said immunoreactive fragments belong to the extracellular region of PDGFrA and/or PDGFrB, more preferably they are comprised in the first 550 amino acids from the NH terminus of the amino acid sequence of the human PDGFrA NP008197, (SWProt P16234, SEQ ID No. 7) and/or PDGFrB NPOO26000 (SWProt P09619, SEQ ID No.8).

The invention will be described in the following non limitative examples.

### FIGURE LEGENDS

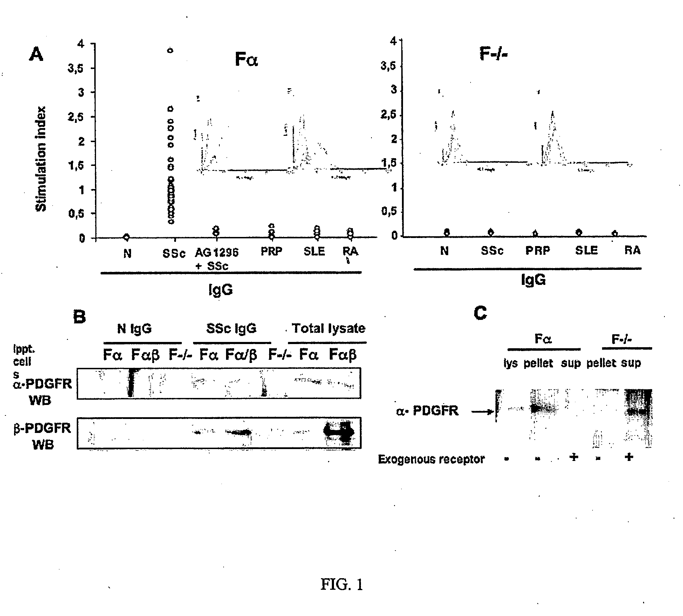

### Fig. 1 Antibodies against PDGF receptor in scleroderma patients

**Panel A.** Levels of reactive oxygen species in Fα and F-/- cells incubated with normal (N; n° 20), scleroderma (SSc; n° 46), primary Raynaud's phenomenon (PRP; n° 15), systemic lupus erythematosus (SLE; n° 15), and rheumatoid arthritis (RA; n° 15), IgG. F cells were also pre-incubated with the selective inhibitor of PDGFR tyrosine kinase (AG 1296). The horizontal bar indicates the mean value. **Panel B.** Fα, Fαβ and F-/- lysates were immunoprecipitated with IgG purified from SSc patients and normal subjects and developed with specific antibodies against the PDGFR α and β subunits (WB). The first two lanes on the right are immunoblots of total proteins. Only SSc IgG efficiently immunoprecipitated PDGFR α and β subunits. Representative results from 1 of 3 experiments are shown. **Panel C.** Scleroderma IgG were incubated with Fα cells, expressing the α chain of PDGFR. Cell lysate was centrifuged and subjected to immunoprecipitation. The supernatant was mixed with the extracellular domain of αPDGFR and immunoprecipitated. The same procedure was carried out with F-/- cells. PDGFR expressed by Fα cells titrated anti PDGFR antibodies from SSc IgG. One experiment representative of 3 is shown.

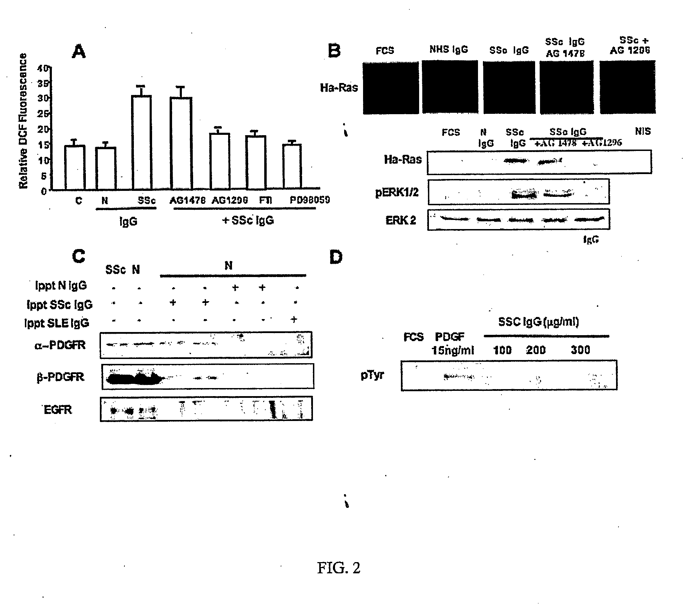

### Fig. 2 SSc Antibodies to αβ PDGF receptor stimulate Ha-Ras-ERK 1/2-ROS signalling in normal fibroblasts

**Panel A.** Levels of reactive oxygen species (mean ± 1 SD), evaluated as DCF fluorescence intensity, in normal human fibroblasts incubated with normal (N; n°= 3) and SSc IgG(n°= 3) (200 µg/ml for 15 min) and pre-incubated with selective inhibitors of EGFR or PDGFR (AG 1478 and AG 1296, respectively) and with FTI-277 and PD98059. **Panel B.** Fluorescence microscopy and immunoblotting for Ha-Ras protein in normal human fibroblasts incubated with normal and SSc IgG (200 µg/ml for 15 min) in the presence and absence of selective inhibitors (AG 1478 and AG 1296 2 µM for 2 h). The slight inhibition on Ha-Ras band by AG1478 was dependent on the high concentration used in this specific experiment (IC₅₀ EGF receptor=3nM). Representative results from 1 of 5 experiments are shown. 40 x Magnification. **Panel C.** Cell lysates were immunoprecipitated with IgG purified from 2 SSc patients, 1 SLE patient and 2 normal subjects and developed with specific antibodies against the PDGFR and EGFR (WB). The first two lanes on the left are immunoblots of total proteins extracted from N or SSc fibroblasts and probed with the antibodies versus the PDGFR and EGFR. One experiment representative of 3 is shown. **Panel D.** Normal human fibroblasts were stimulated with SSc IgG (200 µg/ml for 15 min), PDGF (15 ng/ml for 15 min) or grown in FCS 0.2%. Cell lysates were immunoprecipitated with a polyclonal antibody against PDGFR (subunit β) and the immunoblots were developed with a specific antibody against phosphorylated tyrosine.

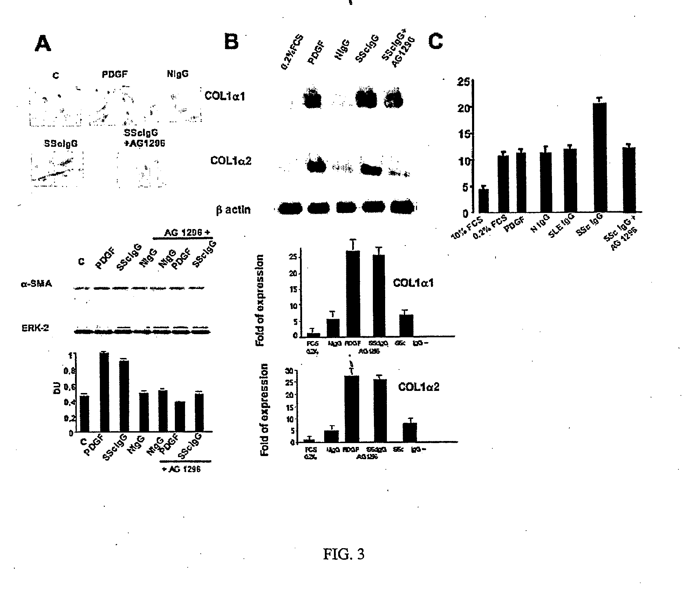

### Fig. 3 Biological Effects of anti PDGFR autoantibodies

**Panel A.** α-SMA protein induction in normal human fibroblasts stimulated with PDGF (15 ng/ml for 24 hours) normal human IgG (NIgG; 200 µg/ml for 24 hours) and scleroderma IgG (SSc IgG; ; 200 µg/ml for 24 hours) in the presence and absence of AG1296 (2 µM for 24 hours). The experiment shown represents a typical blot obtained in 3 independent experiments. Densitometric analysis is shown in the lower part. Data represents the mean value ± SEM of 3 independent experiments. **Panel B.** Type I collagen gene expression by normal human fibroblasts after incubation with 0.2% FCS for 48 hours, PDGF (15 ng/ml for 24 hours) normal human IgG (NIgG; 200 µg/ml for 24 hours) and scleroderma IgG (SSc IgG; ; 200 µg/ml for 24 hours) in the presence and absence of AG1296 (2 µM for 24 hours). Northern blot analysis was used to detect α1(I) and α2(I) collagen mRNA. Representative results from 1 of 3 experiments are shown.

**Panel C** Umbilical vein endothelial cells were incubated with PDGF (15 ng/ml overnight), and with IgG from normal subjects (N), lupus erythematosus systemicus (SLE) and scleroderma (SSc) (200 µg/ml overnight). SSc were also incubated in the presence of AG1296 (2 µM overnight). Apoptosis was then detected by FACS analysis of annexin V-Cys 3 stained cells. Diagram represents the percentage of annexin V-Cys 3 positive cells.

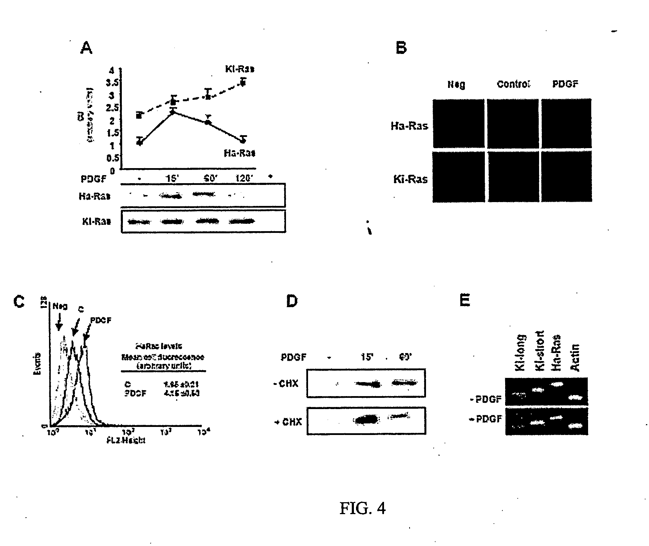

### Fig. 4. Induction of Ras proteins by PDGF in primary fibroblasts

Primary fibroblasts derived from healthy donors (2-5 passages) were incubated in 0.2% of FCS for 48 h and then stimulated with PDGF 15 ng/ml for the indicated time.
**A.** Total proteins were extracted, immunoprecipitated with pan-*Ras* antibodies and immunoblotted with specific antibodies against Ha-*Ras* and Ki-*Ras*. The immunoblot shown is representative of 3 experiments performed in duplicate. DU on the ordinates indicates an arbitrary densitometric unit, normalized to the band of Ha *Ras* without treatment with PDGF set to 1.
**B.** Fluorescence microscopy of fibroblast pre-incubated in 0.2% FCS for 48 h and stimulated with PDGF (15 ng/ml) for 15 min. Cells were stained by indirect immunofluorescence with specific antibodies to Ha-*Ras* and Ki-*Ras*. Representative results from 1 of 5 experiments are shown. Neg indicates immunofluorescence with non-immune sera. 40 x Magnification.
**C.** FACS analysis with anti Ha-*Ras* antibodies of cells treated as indicated in B. The hystograms of a representative experiment are shown. Values are mean ± 1SD of 3 independent experiments performed in duplicate. Both the monoclonal (F235) and polyclonal (SC520) antibodies to Ha-Ras were used with identical results (see Materials and Methods).
**D.** Ha-*Ras4* expression in normal fibroblasts stimulated with PDGF (15 ng/ml) in the presence or absence of cycloheximide (10 µg/ml for 6 h). A representative of 3 experiments is shown.
**E.** Semiquantitative RT-PCR of RNA extracted from cells stimulated with 15 ng PDGF for 60 min. The reactions were carried out as described in materials and methods. Here a representative of 3 reaction (20 cycles) is shown. Under these conditions the intensity of the specific bands was linearly dependent on the concentration of cDNA. Ki long and short represent the two major Ki4B mRNAs differing in the length of 3' UTR.

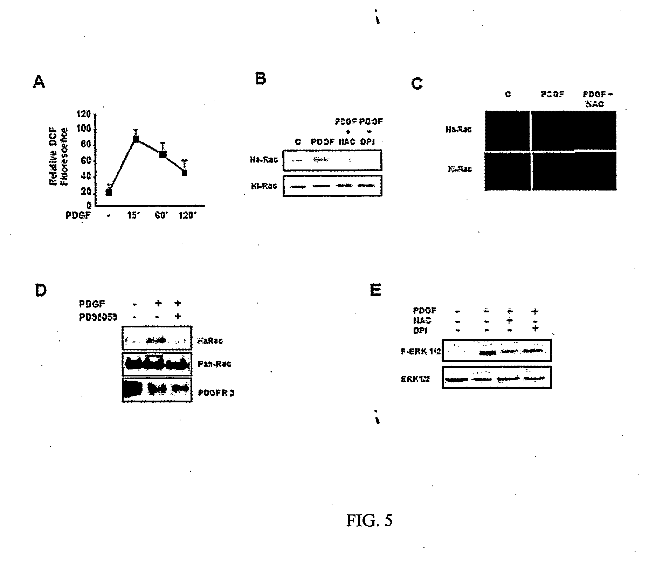

### Fig. 5 ROS induce Ha-Ras in primary fibroblasts

**A.** Time course of ROS induction by PDGF. Primary fibroblasts derived from healthy donors (2-5 passages) were incubated in 0.2% of FCS for 48 h and then stimulated with PDGF for the indicated time. ROS were determined by DCF fluorescence in normal fibroblasts in the presence or absence of PDGF (15 ng/ml). Values represent mean ± 1SD of 5 independent experiments performed in duplicate.
**B.** ROS inhibition abolishes induction of Ha-*Ras* by PDGF. Primary fibroblasts were treated with NAC (20 mM 5 h) and DPI (20 µM for 1 h), treated with PDGF (15 ng/ml for 15 min) and analyzed for Ha and Ki-*Ras* levels as indicated in Fig.1A. A representative of 3 experiments is shown.
**C.** Fluorescence microscopy of fibroblasts pre-incubated in 0.2% FCS for 48 h pre-treated 5 h with NAC (20 mM) and stimulated with PDGF (15 ng/ml) for 15 min. Cells were stained by indirect immunofluorescence with specific antibodies to Ha-*Ras* and Ki-*Ras*. Representative results from 1 of 5 experiments are shown.
**D.** PDGF induction of Ha-Ras is mediated by ERK1/2. Fibroblasts, incubated in 0.2% FCS for 48 h, were treated with the MEK inhibitors, PD98059 (40 µM) 2h, before the treatment with PDGF (15 ng/ml). Ha Ras levels were determined by immuno-precipitation with pan-Ras antibody and immunoblot as indicated in Fig.1A. The extracts were also blotted with anti-PDGF R beta-subunit antibody and pan-Ras antibodies.
E. ROS mediate PDGF induction of ERK1/2. Fibroblasts incubated in 0.2% FCS for 48 h, pre-treated with NAC (20 mM 5 h) and DPI (20 µM for 1 h) were treated with PDGF (15 ng/ml for 30 min) and analyzed for P-ERK1/2 levels as indicated above. A representative of 3 experiments is shown.

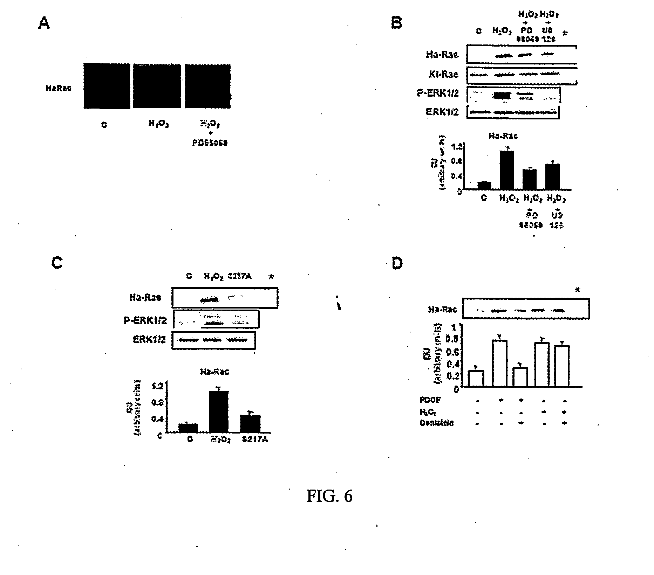

### Fig. 6 ERK1/2 mediate ROS effects on Ras protein.

ROS induction of *Ras* is abolished by the MEK1 inhibitors.
**A.** Fluorescence microscopy of primary fibroblasts with anti-Ha *Ras* antibody, incubated in 0.2% FCS for 48 h and then treated with H₂O₂ (1 µM for 15 min) in the presence or absence of PD98059 (40 µM for 2 h). Representative results from 1 of 3 experiments are shown. 40x Magnification.
**B.** Cells, incubated in 0.2% FCS for 48 h, were treated with the MEK inhibitors, PD98059 (40µM) and U0126 (50µM), for 2 h and 15 min, respectively, before treatment with H₂O₂ (1 mM for 15 min). Immunoblot analysis was carried out with antibodies against total ERK (ERK 1/2) and its phosphorylated forms (P ERK 1/2). Immunoblots were also developed with specific antibodies against Ha-*Ras* and Ki-*Ras* on cell lysates previously immunoprecipitated with a monoclonal anti-pan-*Ras* antibody. Non-immune serum (*) was used as control. One experiment representative of 3 is shown. Densitometric analysis of Ha *Ras* levels is shown in the lower part of the panel. The results of 3 experiments were normalized and represented in arbitrary units as mean ± 1SD.
**C.** primary fibroblasts were transiently transfected with a control or an expression vector carrying the MEK dominant negative variant pBABE-MKK-S217A (C. Marshall, CRC, ICR, UK). Ha-*Ras* and P ERK1/2 levels were determined as indicated. The results of 3 experiments were normalized and represented in arbitrary units as mean ± 1SD.
**D.** Primary fibroblasts were treated with PDGF (15 ng/ml for 15 min) and H₂O₂ (1mM for 15 min) in the presence or absence of genistein (1µg/ml for 1 h). Values are mean ± 1SD of 3 independent experiments performed in duplicate.
**E.** Primary fibroblasts were pre-treated with monensin (MON) (0.1 mM 30 min) or MG132 (0.04 mM 30 min) and treated with PDGF (15 ng/ml for 15 min) and H₂O₂ (1 mM for 15 min). Values are mean ± 1 SD of 3 independent experiments performed in duplicate.

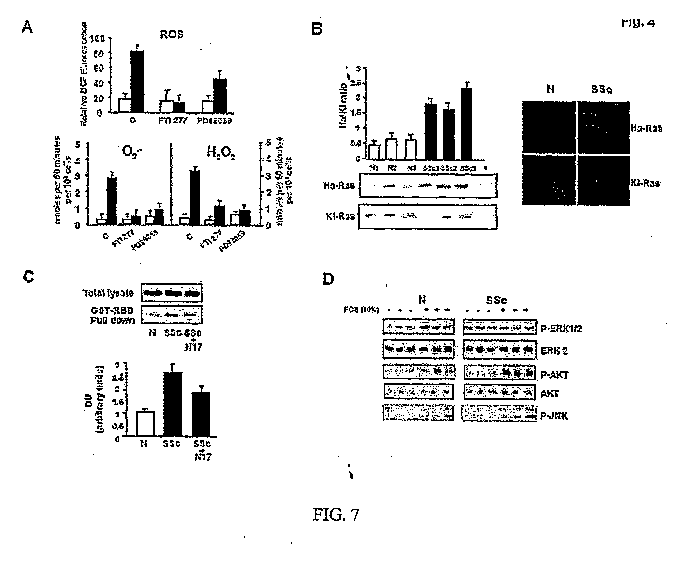

### Fig. 7 Amplification of ROS-Ras signalling in scleroderma fibroblasts.

ROS in primary fibroblasts derived from scleroderma lesions. Reduction of ROS by MEK1 and farnesyl transferase inhibitors
**A.** Levels of reactive oxygen species evaluated as DCF fluorescence intensity (arbitrary units) in 3 normal (25) and in 3 SSc (black) fibroblast cell lines before (48 h in 0.2% FCS) and after incubation with PD98059 (40 µM for 2 h after 48 h in 0.2% FCS) or with farnesyl proteins transferase inhibitors, FTI-277 (20 µM for 2 h) before ROS assay. O₂⁻ , determined by superoxide dismutase-inhibitable cytochrome C reduction assay and H₂O₂ determined by homovanilic acid reduction assay, are shown. Data are the mean ± 1SD of 3 independent experiments performed in duplicate.

### Increased Ha-Ras levels and activity in scleroderma cells

**B. Left Panel.** The data shown were obtained with 3 normal (N) and 3 SSc fibroblast cell lines. Immunoblots were developed with specific antibodies against Ha-*Ras* and Ki-*Ras* on cell lysates, immunoprecipitated with a monoclonal anti-pan-*Ras* antibody. The histogram shows the ratio Ha/Ki derived from 3 independent experiments on the same samples. Non-immune serum was used as control (*). **Right panel.** Fluorescence microscopy of normal and scleroderma fibroblasts incubated in 0.2% FCS for 48 h. Cells were stained by indirect immunofluorescence with anti Ha-*Ras* and Ki-*Ras* antibodies. Representative results from 1 of 3 experiments are shown. 40 x Magnification.
**C.** The upper part of the panel shows the immunoblot of total lysates and GST-RBD bound *Ras* proteins from normal (N) and SSc fibroblasts, probed with anti-pan-*Ras* antibodies. One experiment representative of 3 is shown. SSc fibroblasts were also transfected with the negative Ha-*Ras* variant *Ras*N17. The lower part of the panel shows the densitometric analysis (mean ± 1 SD) of the bands in 3 independent experiments. *Ras* inhibition was not complete, because only a fraction of cells expressed the exogenous transdominant negative *Ras*.
**D.** Immunoblot analysis of total proteins (50 µg) extracted from normal and scleroderma fibroblasts. 3 normal (N) and 3 scleroderma (SSc) fibroblast cell lines were cultured in the absence (0.2% FCS for 48 h) and in the presence of serum (10 % FCS for 15 min after 48 h in 0.2% FCS) before being harvested. Phosphorylated forms of ERK1/2, AKT and JNK were detected by immunoblotting with phosphospecific antibodies. Representative results from 1 of 3 experiments are shown.

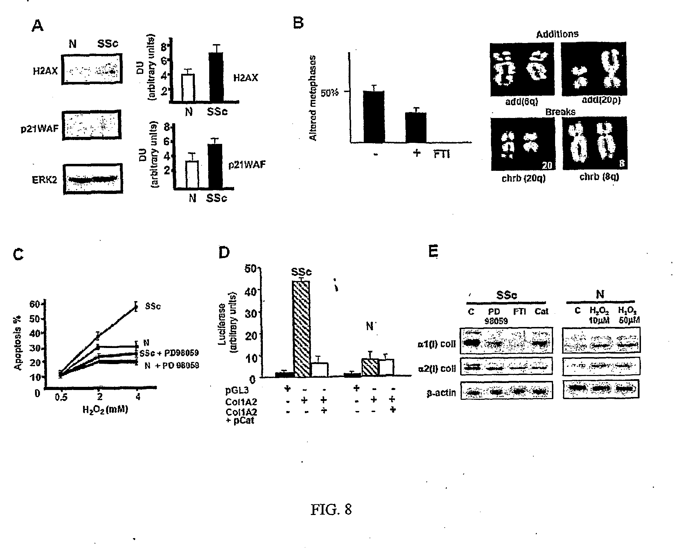

### Fig. 8. Accelerated senescence of scleroderma fibroblasts

**A.** Activation of DNA damage checkpoint in scleroderma fibroblasts. Immunoblot with a specific antibody that recognize phosphorylated histone H2AX or p21WAF. Representative results from 1 of 3 experiments are shown. A polyclonal antibody against ERK 1/2 was used to normalize the amount of protein loaded.
**B.** Chromosomal damage in scleroderma fibroblasts. Karyotype analysis of 3 lines of primary fibroblasts derived from scleroderma lesions. These lines were cultured for 2 passage and treated 48 h with FTI-277 (10 µg/ml). At least 50 metaphases were scored for each line, included 3 normal primary fibroblasts. The frequency of altered metaphases in normal lines was approximately 4%±2 in the presence or absence of FTI (48 h, 10 µg/ml) (data not shown). The aberrations found were not artefact derived from *in vitro* growth of the cells, since they were present in the first passages. Some alterations were more frequent, depending on the time elapsed from the initial passages in culture to the time of analysis. The right panel shows representative chromosomal alterations found in SSc cells.
**C.** Oxidative stress-induced apoptosis in scleroderma cells. Normal (N) and SSc fibroblasts were stimulated for 2 h with increasing concentrations of H₂O₂ in the presence or absence of MEK inhibitor PD 98059 (40 µM). Apoptosis was then detected by FACS analysis of annexin V-C stained cells. Diagram represents the percentage of annexin V positive cells. The data shown are the mean ± 1 SD of 3 independent experiments.
**D.** Activation of ROS-inducible genes in scleroderma cells. Activation of collagen promoter by ROS in SSc cells. N or SSc cells were transfected with the empty vector (pGL3) or with alpha2(I) collagen promoter driving the expression of lucife*Rase* gene (Col1A2) with or without a vectore expressing the human catalase gene (pCat, 10). The ratio of renilla to firefly lucife*Ras*e values was used to normalize co-transfection experiments. Data are expressed as mean ± 1SD (n=3).
**E.** alpha1(I) and alpha2 (I) collagen mRNA after incubation of SSc fibroblasts with PD98059 (40 µM for 24 h), FTI-277 (20 µM for 24 h) or transfection with an expression vector carrying the human catalase gene. Collagen mRNAs in normal cells stimulated with H₂O₂. (for 24 h). Representative results from 1 of 3 experiments are shown.

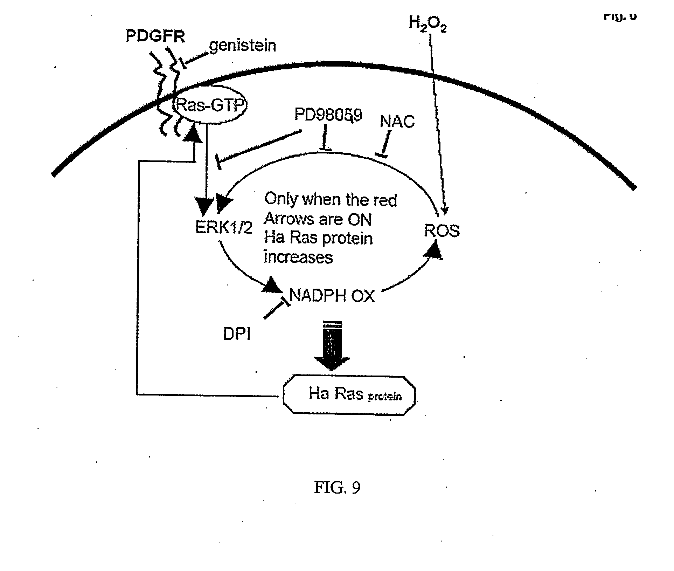

### Fig. 9 Circuitry linking Ras and ROS.

Schematic diagram illustrating the circuitry initiated by PDGF and triggering ROS production by NADPH oxidase. ROS activate ERK1/2, which induce Ha-*Ras*. Once triggered, the circuitry become independent on PDGF signalling. This loop is amplified in scleroderma cells.

### PATIENTS, MATERIALS AND METHODS

### Reagents

Dulbecco's Modified Eagle Medium (DMEM), FCS, L-glutamine, and pen-strept-anfotB solution were obtained from Gibco (Milan, Italy). Recombinant platelet-derived growth factor BB (PDGF-BB) was purchased from Peprotech (Rocky Hill, NJ); FTI-277, farnesyl protein transferase inhibitor H-Ampamb-Phe-Met-OH, and PD 98059, were purchased from Calbiochem (San Diego, CA). Genistein was obtained from ICN Biomedicals (Aurora, OH). Anti Ha-*Ras* (F235 or SC520), anti Ki-*Ras* (F234), anti pan-*Ras* (F132) and anti Rac1 antibodies were purchased from Santa Cruz (CA, USA). Anti phosho-p44/42 MapKinase, anti phospho-SAPK/JNK and anti Akt from Cell Signaling Technology (Beverly, MA). Anti H2AX and anti p21WAF antibodies were obtained from Upstate Biotechnology (Charlottesville, VA); diphenylene iodonium (DPI) from Alexis Biomedicals (Lansen, CH), N-acetyl-L-cysteine (NAC) and cycloheximide from Sigma (St. Louis, MO). U0126 from Promega (Madison, WI) and 2',7'-dichlorofluorescein diacetate (DCFH-DA) from Molecular Probes (Eugene, OR). The following plasmids were employed: dominant negative Ha-*Ras*N17, V12 positive variant of human Ha-*Ras* and V12 positive variant of human Ki-*Ras* (7), dominant negative Rac variant (Rac1N17), dominant positive Rac variant (Rac1V12) (13), dominant negative MEK variant pBabe-MKK-S217A (rat gene bank z30163). The cDNAi for collagen α1(I) (Hf677 clone) and for collagen α2(I) (Hf32 clone) were kindly donated by Dr. Ch M. Lapiere (Laboratorie de Biologie des Tissues Conjonctifs, University of Liege, Belgium).

### Patients

Forty-six consecutive Caucasian patients with scleroderma (8 men and 38 women) with a median age of 58 y (range 35-77) were studied. Diagnosis was made following the ACR preliminary criteria (11) and the patients were classified into the diffuse SSc and limited SSc subset according to LeRoy et al (12). Moreover, patients within the diffuse SSc subset were divided into patients with early disease (2 y or less of disease duration) and patients with late disease ( more than 2 y of disease duration). At the time of the investigation the patients had not received any treatment for the previous 6 weeks. The demographic and clinical characteristics of the study populations are presented in Table 1.

**Table 1:Demographic characteristics of patients and controls**

| Group | N° | M/F | F | Subset | | Median Age Y(range) | Median duration of Raynaud's Phenomenon | Median duration of disease Y(range) |
|---|---|---|---|---|---|---|---|---|
| | | | | ISSc | dSSc | | Y(range) | |
| **SSc** | 46 | 8/38 | | 24 | 22 | 58 | 14 | 7 |
| | | | | | | (35-77) | (2-50) | (<1-48) |
| **PRP** | 15 | 2/13 | | | | 42 | 6 | 6 |
| | | | | | | (22-70) | (2-25) | (2-25) |
| **LES** | 14 | 1/13 | | | | 36 | | 7 |
| | | | | | | (26-50) | | (1-23) |
| **AR** | 15 | 2/13 | | | | 65 | | 11 |
| | | | | | | (22-91) | | (1-28) |

Because Raynaud's phenomenon may precede by years the development of scleroderma, 15 patients with primary Raynaud's phenomenon (PRP) were included in the study (Table 1). Diagnosis of PRP was made according to the criteria reported elsewhere (13). Twenty age, sex and race matched healthy volunteers were also evaluated and constituted the control population. Control groups also included 15 sex and age matched patients with systemic lupus erythematosus and 15 patients with rheumatoid arthritis in whom diagnosis was made according to established criteria (14,15).

After informed consent, a blood sample was taken from patients and controls after acclimatization at 21 °C for 30 min and spun in a refrigerated centrifuge after clot formation. The supernatants were collected and stored at -30°C until the assay, usually within 4 weeks.

### Cell Lines

Mouse embryo fibroblasts derived from PDGF receptor knock-out embryos that do not express α and β chains PDGFR subunits (F-/- cells) and F-/- cells infected with PDGFR α and β subunits (F α; F β; F αβ) have been described elsewhere (16)

### Primary Fibroblast cultures

Human skin fibroblasts were obtained from punch biopsies taken from the forearms of normal volunteers and from the involved skin of patients who fulfilled the preliminary criteria of the American Rheumatism Association for the diagnosis of Systemic Sclerosis as described (14) Fibroblasts between the fourth and the sixth sub passage were used for all experiments.

### Purification of IgG

IgG fractions were purified by affinity chromatography on protein A/G-sepharose column (Pierce, Rockford, IL) from serum of normal subjects, of scleroderma and systemic lupus erythematosus patients, and from patients with rheumatoid arthritis. Protein concentrations were estimated by spectrophotometry. All preparations were endotoxin-free as determined by Limulus amoebocyte assay.

### Bio-assay for anti PDGFR autoantibodies

Patients and control serum samples were tested for the presence of PDGFR activating autoantibodies in a functional bioassay based on the production of ROS by mouse embryo fibroblasts infected with PDGFR α and/or β subunits and exposed in vitro to immunopurified IgG. Control cells were F-/- cells devoid of PDGFR. In brief, cells were plated in duplicate at a density of 30,000 cells in 1.83-cm² wells and incubated for 24 hours at 37°C in 0.2 percent fetal calf serum. The cells were then washed with phosphate-buffered saline and incubated with a pre-determined amount of control or patient IgG for 15 minutes at 37 °C before ROS production determination.

Fluorimetric determination of intracellular ROS generated by adherent fibroblasts was estimated after loading the cells with 2',7'-dichlorofluorescein diacetate (DCFH-DA 10 µM, Molecular Probes, PoortGebouw, The Nederlands) for 15 min at 37° C. The DCF fluorescence intensity was measured by a CytoFluor plate reader (PerkinElmer, Wallac, Finland) (excitation wavelength, 485nm; emission wavelength, 530 nm) (8). Ten measurements were made in each single well from ten distinct points and the values averaged. Each IgG sample was run in duplicate and the average has been recorded. The results are expressed as DCF fluorescence intensity calculated subtracting from the DCF fluorescence intensity of the test IgG the DCF fluorescence intensity of a negative control obtained by cell cultured without IgG. The intra-plate and the inter-plate coefficient of variation was less than 3 %. The samples were recorded as positive if the SI was greater than the mean plus three standard deviations of the normal group.

In selected experiments ROS generation was evaluated after the addition of PDGF receptor tyrosine kinase inhibitor (AG 1296; 2 µM for 2 hours), Epidermal growth factor (EGF) receptor tyrosine kinase inhibitor (AG 1478; 2 µM for 2 hours), a chemical inhibitor of ERK 1/2 signalling (PD 98059; 40 µM for 2 hours), and a farnesyl protein transferase inhibitor to block Ras farnesylation (FTI-277; 20 µM for 2 hours). AG 1296, AG 1478, PD 98059 and FTI-277 were obtained from Calbiochem (San Diego, CA).

### Cell lysis and immunoblotting

Cell culture plates were lysed with 0.3 ml of cold RIPA buffer (1x PBS, 1% Nonidet P-40, 0.5% sodium deoxycholate, 0.1% SDS, 2 mM sodium orthovanadate, 2 µg/ml aprotinin, 1 mM PMSF) and processed for immunoblotting as described (8).

### Immunoprecipitation assays

PDGF receptor was immunoprecipitated from cultured cells with 200 µg of IgG. Immunocomplexes were isolated, subjected to electrophoresis, and immunoblotted with anti PDGFR α and β subunits antibodies (Santa Cruz) and revealed by chemiluminescence (Amersham, Sweden).

Ras proteins were immunoprecipitated from cultured fibroblasts with polyclonal anti pan Ras antibody (Santa Cruz Biotechnology, Santa Cruz, CA) following recommended procedures from the manufacturer. Immunocomplexes were isolated, subjected to electrophoresis, and immunoblotted with anti Ha-Ras antibodies and revealed by chemiluminescence (Amersham, Sweden).

### Immunoblotting

Cell culture plates were lysed with 0.3 ml of cold RIPA buffer (1x PBS, 1% Nonidet P-40, 0.5% sodium deoxycholate, 0.1 % SDS, 2 mM sodium orthovanadate, 2 µg/ml aprotinin, 1 mM PMSF) and processed as described (14).

### Absorption of autoantibodies with recombinant PDGF receptor

Scleroderma IgG (200 µg/ml) were incubated for 2 hours at 4 °C with recombinant PDGFR expressed by Fα cells. This mixture was then centrifuged (14,000 g for 15 minutes at 4°C) and the supernatant was again subjected to immunoprecipitation with the extracellular domain of PDGFR α subunit (R&D Systems, Wiebaden, Germany), as antigen source, to test whether the PDGFR band had been removed. F-/- cells were used as control cells. Immunoblot were challenged with anti PDGFR α and β subunits antibodies (Santa Cruz) and revealed by chemiluminescence (Amersham, Sweden).

### Immunofluorescence

Fibroblasts, cultured on Lab-Tek chamber glass slides (Nalge-Nunc, IL, USA) and starved 48 h before stimulation or addition of inhibitors, were fixed 4% paraformaldheide, permeabilized by 0.1% Triton-X, and stained with a monoclonal antibody against Ha-Ras and then with a tetramethylrhodamine-isothiocyanate (TRITC) conjugated secondary antibody (Molecular Probes, NL). Slides were mounted with Vectarshield (H-100; Vector, Burlingame, CA) and examined using a BioRad (Hemel Hempstead, UK) microradiance confocal laser-scanning microscope equipped with argon and helium/neon lasers. Acquired images were analysed using the Laser Sharp Processing BioRad software (version 3.2). All images from different slides condition were acquired in double-blinded fashion.

### RNA isolation and Northern analysis

Total cellular RNA was extracted using the Rneasy Mini kit (Qiagen, Hilden, Germany). Ten micrograms total RNA was then used for Northern blot analysis following a described procedure (7).

### Apoptosis assay

Endothelial cells will be isolated from umbilical cord. Cells were stimulated with SSc IgG (200µg/ml), normal IgG (200µg/ml) and PDGF (15 ng/ml) for 12 hours in the presence and in the absence of inhibitors of PDGFR and EGFR tyrosine kinase activity.

Normal and scleroderma fibroblasts were grown to 80-90% confluence and treated for 2 h with different concentrations of H₂O₂. When needed, cells were preincubated 30 min with PD 98059 (40 µmol/L).

Six hours after the removal of the stimulus, apoptosis was detected by FACS analysis using annexin V-Cy3 (Clontech, Palo Alto CA).

### Statistical analysis

Data are expressed as mean ± 1SD. Mean values were compared using Student's paired and unpaired t-test. P values less than 0.05 were considered significant. All values are 2-tailed.

### Flow cytometric analysis with anti HaRas antibody

Cells were grown to semiconfluency in 60mm culture dishes. After trypsin detachment, 5x10⁵ cells were suspended in 1 ml of phosphate buffered saline (PBS) and fixed overnight with 1% formaldehyde at room temperature. Next, cells were permeabilized with 0.1% TritonX100 for 40 min at 4°C, washed 4 times with 2 ml of PBS containing 2% FBS, 0.01% NaN₃, 0.1% TritonX100 (buffer A), and incubated for 45 min at 4°C with 1:50 dilution of monoclonal and polyclonal anti Ha*Ras* antibodies (Santa Cruz Biotechnology, Santa Cruz, CA, USA). The cells were then washed twice with the same buffer and incubated for 45 min at 4°C with Cy2-conjugated anti-mouse IgG antibodies (Amersham Pharmacia Biotech, Milano, Italia) at 1:50 dilution. Control cells were incubated with Cy2-conjugated anti mouse IgG antibodies alone. After two washes in buffer A, cells were resuspended in PBS and analyzed by flow cytometry using FACScan (BD, Heidelberg, Germany) and WinMDI software.

### ROS determination

Fluorimetric determination of intracellular ROS generated by fibroblasts was estimated after loading the cells with DCFH-DA (10 µM) for 15 min at 37° C before assessing DCF fluorescence level (15). Superoxide anion release was estimated using the superoxide dismutase-inhibitable cytochrome c reduction (12). H₂O₂ release from fibroblasts into the overlying medium was assayed using a modification of the method of Valletta and Berton (1987) (16). Oxidative activity imaging in living, transfected cells was evaluated as described (12, 17).

### Ras activation assay

Cells were washed in ice cold PBS and lysed with 0.5 ml per plate of lysis buffer (20 mM Hepes, pH 7.4, 1% NP40, 150 mM NaCl, 10 mM MgCl₂, 10% glycerol, 1 mM EDTA, 1 mM Na vanadate, 10 mg/ml leupeptin and 10 mg/ml aprotinin). Lysates were cleared by centrifugation (13,000 rpm at 4°C) and diluted to 1 mg/ml with lysis buffer.

GST-RBD expression in transformed Escherichia coli was induced with 1mM IPTG for 1-2 h and fusion protein was purified on glutathione-Sepharose beads. The beads were washed in a solution containing 20 mM Hepes, pH 7.4, 120 mM NaCl, 10% glycerol, 0.5% NP40, 2 mM EDTA, 1 mM Na vanadate, 10 mg/ml leupeptin and 10 mg/ml aprotinin. For affinity precipitation lysates were incubated with GST-RBD pre-bound to glutathione-Sepharose (30 ml packed beads) for 60 min at 4°C with rocking. Bound proteins were eluted with SDS-PAGE sample buffer, resolved on 12% acrylamide gels and subjected to Western blotting. Blots were probed with anti *Ras*, clone *Ras* 10 (Upstate Biotechnology).

### Transfection

For transfection experiments, confluent fibroblasts were plated in 100 mm dishes in culture medium. After 24 h, the medium was discarded, replaced with fresh culture medium and the cells transfected. Transfection experiments were carried out in duplicate using a liposomal method (Effectene, Qiagen, Hilden, Germany).

In selected experiments the recombinant plasmid pGbC1A2-P obtained by cloning the promoter of human type I collagen α2 chain gene (COL1A2) (20) was cotransfected with either PS3CAT carrying human catalase gene (a kind gift of Dr. Irani The Johns Hopkins, Baltimore) or a control vector following the procedure described above. The lucife*Rase* activities of the samples were measured with a TD-20/20 luminometer (Turner Design) and the ratio of Renilla to firefly lucife*Rase* values was used to normalize the co-transfection experiments.

### RT-PCR of Ha- and Ki-Ras mRNA

Total RNA and cDNA synthesis was performed as described (18).Two µl of cDNA products (derived from 2.5 µg of total RNA) were amplified with 1 unit of Ampli Taq Gold (PE Applied Biosystems) in the buffer provided by the manufacturer which contains no MgCl₂, and in the presence of the specific primers for Ha-, Ki-*Ras* and actin genes (see below). The amount of dNTPs carried over from the reverse transcription reaction is fully sufficient for further amplification. Reactions were carried out in the Gene Amp PCR system 9600. A first cycle of 10 minutes at 95°C, 45 seconds at 65°C and 1 minute at 72°C was followed by 45 seconds at 95°C, 45 seconds at 65°C and 1 minute at 72 °C for 30 cycles. The conditions were chosen so that none of the cDNAs analyzed ; reached a plateau at the end of the amplification protocol, i.e. they were in the exponential phase of amplification, and that the two sets of primers used in each reaction did not compete with each other. Each set of reactions always included a no-sample negative control. We usually performed a negative control containing RNA instead of cDNA to rule out genomic out genomic DNA contamination. The following primers were used:
Ki-*Ras* long, left primer: ACATCTCTTTGCTGCCCAAT; SEQ ID No. 1
right primer: GAGCGAGACTCTGACACCAA; SEQ ID No. 2.
Ki-*Ras* short, left primer: TCGACACAGCAGGTCAAGAG; SEQ ID No. 3
right primer: AGGCATCATCAACACCCTGT. SEQ ID No. 4
Ha-*Ras*, left primer: CCAGCTGATCCAGAACCATT; SEQ ID No. 5
right primer: AGGTCTCGATGTAGGGGATG. SEQ ID No. 6

### Cytogenetic analysis

Cytogenetic studies were performed on fibroblasts before and after a 24 h incubation with FTI-277 (20 µM). Fibroblasts were cultured on cover glasses placed in a 35 mm Petri dish. After adding 2 ml of Chang Medium® B the dishes were incubated for 48 h in a 5% CO2 incubator at 37°C and the cultures harvested after adding Colcemid solution (0,1 µg/ml) for 90 minutes. The cover glasses were fixed and Q-banded following standard procedures. The evaluation was performed using a fluorescence microscope Zeiss Axioplan 2. The images were captured with a couple-charged camera device connected to a personal computer running MacKtype 5.4 software (Powergene Olympus Italy). Chromosome identification and karyotype designation were made following the criteria of the International System for Human Cytogenetic Nomenclature (ISCN).

### Chromatin extraction and H2AX phosphorylation

Cell culture plates were lysed with 0.2 ml buffer (120 mM NaCl, 40 mM Hepes, 5 mM MgCl2, 1 mM EGTA, 0.5 mM EDTA, 0.6% Triton X 100 , 2 mM sodium orthovanadate, 2 µg/ml aprotinin, 1 mM PMSF) and centrifuged at 14000x g for 15 min at 4°C and the protein content was measured with the Bio-Rad Protein assay (12). The pellet was resuspended in 40 µl of buffer with 80-100 U of Dnase, and incubated for 15 min on ice. Extracts were denatured and resolved in 12% SDS PAGE. Immunoblot with specific antibodies was carried out as described above.

### Statistical analysis

Data are expressed as mean ± 1 SD. Mean values were compared using Student's paired and unpaired t-test. P values less than 0.05 were considered significant. All values are 2-tailed.

### RESULTS

### Immunoglobulins from scleroderma patients induce ROS and react with PDGF receptor.

*In vitro* SSc fibroblasts spontaneously release ROS (7) and IgG derived from SSC patients bind normal human fibroblasts (10). Since PDGF induces accumulation of ROS (9) the authors investigated if agonistic serum antibodies targeting PDGFR may be present in SSc patients. To test this hypothesis, they purified total IgG from systemic sclerosis patients and determined their biological activity by measuring : 1. ROS levels; 2. tyrosine phosphorylation of PDGFR: 3. ERK1/2 activation in the presence or absence of specific PDGFR inhibitors. As target cells they made use of a mouse embryo cell line, carrying inactive copies of α and β PDGFR subunits, as reference. The same line, expressing recombinant α or β PDGFR subunits (Fα, Fβ and Fαβ) was challenged with the various IgG fractions. Cells were starved and incubated with the peroxide-sensitive fluorophore DCF prior to treatment with purified IgG to determine ROS.

SSc IgG stimulated ROS production in Fα, Fβ and Fαβ in a dose-dependent manner. ROS rapidly increased to its maximum level 15 minutes following IgG addition and returned to the baseline 40-120 min. The best discrimination between normal and SSc IgG was obtained with Fα cells and using approximately 200 µg/ml of IgG. These conditions were followed in all subsequent experiments unless otherwise specified.

The authors next tested the prevalence of stimulatory IgG in a group of 46 unrelated scleroderma patients. Figure 1A shows that the levels of ROS induced by SSc IgG (200 µg/ml for 15 minutes per 20000 cells; 168.8 ± 59) were significantly higher (P< 0.0001) than the ROS generated following the incubation with normal IgG (1.4± 2.9), PRP IgG, (5.1±7), SLE IgG (4.8±9), and RA IgG (2.7±7). Using the 95 percentile as the upper limit of normal values, antibodies stimulating ROS levels were found in all scleroderma patients and in none of the normal subjects (Fig.1A). Antibodies were not detected in patients with primary Raynaud's phenomenon (PRP), as well as in patients with systemic lupus erythematosus (SLE) and with rheumatoid arthritis (RA). ROS-inducing activity of the SSC IgG was mediated by PDGFR. This is shown by : 1. ROS induced in Fα cells incubated with SSc IgG was inhibited by pre-incubating the cells with the PDGFR tyrosine kinase inhibitor AG 1296 (2µM for 2 hours) (Fig 1A); 2. SSc IgG did not stimulate ROS in F-/- cells (Fig 1A); 3. SSc IgG but not IgG from normal subjects immunoprecipitated PDGFR α and β subunits from PDGFR-expressing cells (Fig 1B); 4. PDGFR-interacting antibodies, derived from of scleroderma IgG, were completely removed by pre-absorption with Fα cells (Fig.1C). Conversely, pre-absorption with F-/- cells was unable to remove PDGFR interacting antibodies (Fig.1 C). Moreover, the supernatant following the adsorption with Fα cells did not stimulate ROS production (data not shown).

### IgG derived from sclerodermia patients trigger Ras-ERK1/2-ROS cascade in normal fibroblasts

To dissect the signalling cascade triggered by IgG derived from SSc patients, the authors analysed the ROS-generating activity of SSc IgG (3 patients versus 3 normal controls) in the presence of specific inhibitors: 1. an inhibitor of EGFR signalling (AG1478, 2 µM for 2 hours); 2. a farnesyl transferase inhibitor, FT-277 (20 µM for 2 hours), required fro Ras attachment to the plasma membrane: 3. a MEK inhibitor, the kinase located upstream ERK1/2, PD98059 (40 µM for 2 hours). These inhibitors prevented ROS induction by SScIgG in normal fibroblasts (Fig2A). Conversely, these inhibitors significantly reduced ROS levels in fibroblasts derived from sclerodrma lesions (8). To get insights on the specific signalling cascade triggered by SSc IgG via PDGFR, the authors analysed the biological activity of Ras in SSc cells or in normal cells challenged with SSc IgG. They found that both PDGF and SSc IgG induced in primary cells Ras protein levels. This effect was ERK1/2 dependent and specific of primary cells. Fig.2B shows that IgG from SSc patients induced Ha-Ras and this induction was prevented by PDGFR inhibitor, as indicated by immunofluorescence with specific antibodies and immunoblot.

To determine more precisely the target of SSc IgG, the authors immunoprecipitated IgG from 2 SSc patients, 1 SLE patients and 2 normal subjects at the same protein concentration (200 µg/ml). The immunoprecipitates were separated on PAGE and immunoblotted with *bona fide* PDGFR α and β subunits and anti EGFR antibodies. Figure 2C shows that only IgG isolated from SSc patients efficiently immunoprecipitated PDGFR α and β subunits. IgG isolated from serum of SSc patients did not recognize recombinant α and β subunits of PDGFR by direct immunoblot, indicating that these antibodies recognize conformation(s) present only in the native receptor (data not shown).

Furthermore, to determine the stimulatory nature of antibody-receptor interaction, the authors challenged normal fibroblasts with increasing concentrations of IgG derived from 1 SSc patient for 15 min and tested tyrosine phosphorylation of the PDGFR. IgG derived from a SSC patient induced in a dose-dependent manner tyrosine phosphorylation of the PDGFR (Fig. 2D). They noticed that IgG derived from several SSc patients (approximately; 4) induced prolonged, albeit less intense, tyrosine phosphorylation of PDGFR compared to PDGF (data not shown).

### Biological consequences of anti-PDGFR agonistic antibodies derived from SSc patients

To determine the biological effects induced by SSc IgG, the authors assayed the expression of 2 genes, that characterizes fibroblasts in systemic sclerosis patients : α-smooth muscle actin (α-SMA) and type I collagen, in normal human fibroblasts exposed to SSC IgG. Actin (α-SMA) is a distinctive marker of myofibroblasts, mesenchymal cells which develop from fibroblasts and possess characteristics of both smooth muscle cells and fibroblasts. Human fibroblasts were stimulated with PDGF (15 ng/ml for 24 hours) normal human IgG (NIgG; 200 µg/ml for 24 hours) and scleroderma IgG (SSc IgG; ; 200 µg/ml for 24 hours) in the presence and absence of AG1296 (2 nM for 24 hours) before immunohistochemical analysis and immunoblotting to detect α-SMA. Actin (α-SMA) was stimulated by SSc IgG and not by normal IgG (Fig.3A). Type I and type II collagen mRNAs were robustly induced by SSc IgG and not by normal IgG (Fig.3B).

Finally, the authors tested the sensitivity of endothelial cells to SSc IgG induced apoptosis When SSc cells were stressed and subjected to SSC IgG (200µg/ml overnight), they were more prone to apoptosis than control cells. Apoptosis was prevented by incubating the celsl with a PDGFR inhibitor. Note that under the same conditions, PDGF was unable to induce apoptosis (Fig.3C).

### DISCUSSION

### Stimulatory antibodies to the PDGF receptor in systemic sclerosis

The present study documents the presence of PDGFR stimulatory antibodies in the serum of patients with scleroderma (SSC). This conclusion is supported by 5 independent experimental evidences: 1. purified immunoglobulin fractions from SSC patients induced ROS levels in PDGFR+, not PDGF-/-, cells; 2. immunoglobulin fractions from SSC patients recognized and immunoprecipitated PDGFR (α and β chains) in its native configuration; 3. the PDGFR-binding and the ROS-generating activities of SSC IgG were removed by pre-adsorption to PDGFR-expressing cells and not to PDGF-/- cells; 4. immunoglobulin fractions derived from SSC patients induced myofibroblast conversion and collagen type I and ROS production in normal fibroblasts; 5. last, but not least, the authors have found these antibodies in all systemic sclerosis patients analysed so far (46) and never in normal (20) controls. ROS-inducing activity of the SSC immunoglobulins was inhibited by PDGFR inhibitor(s). These antibodies were not detected in patients with primary Raynaud's phenomenon, Systemic Lupus Erythematosus and Rheumatoid Arthritis. They found, though, antibodies with the features indicated above, selectively in patients presenting graft-versus-host disease (GVHD) with scleroderma-like skin lesions after allogeneic bone marrow transplantation. Based on these indications, the presence of these antibodies is disease-specific and it marks systemic sclerosis patients.

### Antibodies, active PDGF-R and ROS

The onset of the systemic sclerosis may be triggered by the accumulation of these antibodies in the blood. The authors have identified the cascade initiated by PDGF and these antibodies and they have discovered a novel regulation of Ras proteins by growth factors and ROS. In normal primary cells, Ras protein levels - are maintained low by continuous degradation. PDGF induces transiently ROS, which stimulate ERK1/2, that ultimately prevent Ras degradation by the proteasome. ROS inhibit these signals via ERK1/2. PDGF by increasing ROS levels stabilize Ras protein (8).

Both PDGF and the antibodies from SSC patients induce ROS and stabilize Ras (Fig. 2B). However, they display important differences in terms of kinetics of receptor activation. The antibodies are long acting PDGFR stimulators because their effect is long lasting compared to PDGF. ROS induced by SSC IgG last 60-120 min, whereas PDGF-induced ROS return to baseline in 15-30. Persistent ROS accumulation maintains Ras-ERK1/2-ROS higher than normal controls and it may explain higher ROS levels in SSC cells in low serum *in vitro.* Although the relative tyrosine phosphorylation induced by SSC IgG was lower than PDGF, it lasted longer (60 versus 15 min) (Fig. 2D and data not shown).

Antibodies to PDGF receptor may remain longer in the membrane and generate a persistent, albeit less intense, stimulus compared to PDGF.

### Early and late Systemic sclerosis phenotypes

The authors have analysed the short and long term biological effects of SSc IgG in normal cells. The phenotypes closely replicate the features of systemic sclerosis *in vivo.* High ROS levels induced by SSC IgG, render fibroblasts and endothelial cells more responsive to serum and other growth factors, due to the high levels of Ha Ras protein and active ERK1/2. Activation of transcription of collagen genes and actin (α-SMA) results in the appearance of myofibroblasts and fibrosis. On the other hand, endothelial cells become more prone to apoptosis when stressed by ROS. The authors have noted, however, a phenotype in cells exposed to ROS and IgG SSc that may explain the long term consequences of the disease. Fibroblasts derived from SSC patients undergo rapid senescence and accumulate DNA and chromosomal aberrations (8). This may explain the loss of cells in chronic lesions. Our data provide a mechanistic explanation of the scleroderma phenotype. The epitope on PDGFR recognized by the autoantibodies could be relevant for a more sensitive and less cumbersome diagnostic test, as well as to develop target specific therapies and to explain the clinical differences of scleroderma patients in case different epitopes on PDGFR were engaged by the autoantibodies.

The expert in the field shall appreciate that the identification of epitopes could be performed by many methods known on the art as for example, phage display libraries. Autoimmunity may be induced by epitopes exposed as a result of a prolonged injury of vascular endothelial cells, since endothelial cell damage is an early event in the clinical history of scleroderma. In this scenario anti PDGFR autoantibodies follow a primary, distinct event although pathogenically relevant for the persistence of the vascular and fibrotic features. Alternatively, it can be assumed that the autoantiboby is the primary event. The answer to this question would probably come from longitudinal studies of patients with Raynaud's phenomenon who end developing overt scleroderma. In any case, the absence of the autoantibody in our patients with primary Raynaud's phenomenon suggests it has no role in the pathogenesis of this vasospastic disorder and its detection could be exploited to distinguish patients with primary Raynaud's phenomenon from patients with scleroderma.

The most frequent autoantibodies associated with scleroderma include anti centromere and antitopoisomerase-I antibodies, found in 20 to 30% and in 9 to 20%, respectively (28). Although they may be of help in diagnosis and prognosis for identifying groups of patients at risk for specific clinical manifestations, it remains controversial at best whether these autoantibodies have an actual role in pathogenesis.

Within the large array of autoimmune diseases, only Grave's disease, an organ-specific autoimmune disease, is characterized by a stimulatory autoantibody against a (TSH) receptor. Ours is the first report of a similar finding in a connective tissue disease. Furthermore, our data put humoral immunity and the Th2-dependent immune response back to the core of pathogenesis of scleroderma (29), in agreement with the analysis of gene expression in scleroderma skin (30).

In conclusion, the authors have identified antibodies against the PDGF receptor in patients with scleroderma, endowed with agonistic activity. These antibodies trigger an intracellular loop involving Ha-Ras- ERK 1-2- ROS, which leads to endothelial cell injury and increased collagen gene expression. Recently, the authors have been successful in purifying the anti-PDGFR ROS stimulating antibodies and test their biological activity as purified clones. This strongly argues for a causal role in the onset of systemic sclerosis. One important implication derived from the data shown here is the identification of possible tools for diagnosis and targeted therapy of systemic sclerosis.

### PDGF and Reactive Oxygen Species (ROS) regulate Ras protein levels in primary human fibroblasts via ERK1/2

### RESULTS

### Induction of Ras protein levels by PDGF

In quiescent primary human fibroblasts Ha-Ras protein was almost undetectable. To determine precisely the levels of Ki and Ha Ras protein, the authors immunoprecipitated total cell proteins, solubilized in 0.1% SDS (RIPA buffer), with pan Ras antibody. The immunoprecipitates were separated by gel electrophoresis and blotted with specific Ras isoform antibodies. Stimulation of the cells with PDGF for 15 min was sufficient to induce Ha-Ras protein, which decreased to the initial levels, 120 min after stimulation, notwithstanding the presence of the growth factor. Ki-*Ras* protein levels were also stimulated by PDGF but less efficiently and with a different kinetics. Ki-*Ras* was poorly induced and decayed slowly (3 h of PDGF stimulation) (Fig 4A). Ha-*Ras* induction was also visible by fluorescence microscopy (Fig.4B) or FACS analysis with anti-Ha-*Ras* monoclonal or polyclonal specific antibodies (Fig.4C).

Treatment of the cells with the translational inhibitor cycloheximide did not prevent Ha-*Ras* induction by PDGF (Fig.4D). Moreover, PDGF treatment did not change mRNA levels of Ki and Ha-*Ras* (Fig.4E). To rule out that Ha-Ras induced by PDGF was associated to lipid-rich membrane fractions and was not efficiently extracted by immunoprecipitation procedures, the authors treated the cells with 50 mM cyclodextrin to deplete cholesterol before immunoprecipitation. Treatment with cyclodextrin did not abolish PDGF induction of Ha-Ras (Fig. 4 Supplementary Material).

Taken together, these data indicate that the levels of Ha and Ki-*Ras* proteins are post-translationally regulated by PDGF in primary fibroblasts. The authors estimated from several experiments that the half-life of Ha-Ras protein induced by PDGF was approximately 40 min (Fig. 4A and data not shown).

### PDGF stimulation of ROS and ERK1/2

PDGF activates ROS production by stimulating NADPH oxidase (3a, 19a) (Fig.5A). The kinetics of ROS production following PDGF stimulation replicated that of Ha *Ras* induction (Fig.4 and Fig.5A). To determine if ROS were involved in Ha-Ras induction by PDGF, the authors treated the cells with a non-specific ROS scavenger (N-acetyl-cysteine, NAC) or NADPH oxidase inhibitor (DPI) and measured Ha-*Ras* levels in the presence or absence of PDGF. Figure 5B shows that NAC and DPI significantly inhibited PDGF induced Ha-*Ras* levels. This is also shown by immunofluorescence with anti Ha-*Ras* antibodies. (Fig. 5C). The kinetics of Ha-*Ras* induction by PDGF in normal cells replicated also ERK1-2 activation profile (data not shown), suggesting a relation between MEK-ERK1/2 and induction of Ha-*Ras*. To get an insight into this process, they measured Ha-*Ras* in cells pre-treated with a chemical inhibitor of ERK 1-2 signalling (PD 98059), which inhibits MEK (MAPKK), a kinase located upstream of ERK 1/2 MAPK. Treatment of the cells with PD98059 inhibited Ha Ras induction by PDGF (Fig. 5D). In the same extracts, subjected to immunoprecipitation with anti-Ras antibodies, they measured PDGF receptor. Figure 2D shows that PDGF treatment down-regulated the receptor, independently on ERK1/2 activation (Fig. 5D). To determine if ERK1/2 activation by PDGF was also sensitive to ROS depletion, they treated the cells with PDGF in the presence of a general ROS scavenger (NAC) or the NADPH oxidase inhibitor (DPI). Fig. 5E shows that ERK1/2 activated by PDGF was sensitive to NAC and DPI, indicating that ROS depletion interfered with ERK1/2 activation by PDGF. Time course experiments indicated that ROS were not required for early activation of ERK1/2 (5 min of PDGF stimulation). They, however, amplified MEK-ERK1/2 activities 15-20 min after PDGF (Fig. 5E).

### ROS induce Ha Ras level

The data presented above indicate that PDGF *via* ROS and ERK1/2 induce Ha-Ras protein levels in primary fibroblasts. To investigate the mechanism and the link between PDGF and ROS, the authors stimulated the cells with H₂O₂ in the presence of chemical and biological inhibitors of ERK 1-2 signalling. To this end, they employed 1. PD 98059 and U0126 (2 h and 15 min incubation, respectively), which inhibit MEK (MAPKK); and 2. a dominant negative MEK variant that inhibits cellular MEK (see Materials and Methods). The results, shown in Fig. 6A, 6B and 6C, demonstrate that induction of Ha-*Ras* by H₂O₂ was abolished by MEK inhibition. To confirm that H₂O₂ was downstream PDGF, they treated the cells with genistein, a tyrosine kinase inhibitor, in the presence of PDGF or H₂O₂. The data shown in Fig. 6D indicate that H₂O₂ was a powerful inducer of Ha *Ras*, also in the presence of genistein. As expected, the drug inhibited the induction of Ha-*Ras* by PDGF. However, longer incubation periods (90 min) in the presence of genistein, inhibited Ha-Ras and ERK1/2 induced by H₂O₂, indicating that long term effects of H₂O₂ required active PDGF receptor (data not shown). Taken together the data, illustrated in Fig. 5 and Fig. 6, indicate that PDGF *via* ERK1/2 and *via* ROS induced Ha-Ras protein. ROS are downstream the PDGF receptor, since they induced Ha-Ras independently on the activation of the receptor. However, Ha Ras protein induced by ROS was transient in the absence of active PDGF receptor (data not shown). Down-regulation of ERK1/2 (Fig. 5D, 5E and Fig. 6) reduced Ha Ras levels. Maintaining ERK1/2 high, by expressing constitutive active Ha-*Ras* or a dominant positive MEK protein, ROS production was high and endogenous Ha Ras did not decay (data not shown). This process was specific to Ha-*Ras*, since Ki-*Ras* levels were only marginally affected by ERK1-2 inhibition in primary fibroblasts. More importantly, Ha-*Ras* stabilization was peculiar to primary cells, since stabilized cells such as 3T3 fibroblasts, CHO, PC12 and COS7 contained stable and high levels of Ha-*Ras*, which were insensitive to ERK1/2 inhibition (data not shown).

The data shown above do not clearly indicate the mechanism responsible for Ha Ras stabilization induced by PDGF-ROS-ERK1/2. To this end, the authors treated the cells with MG132, a widely used proteasome inhibitor and monensin, a toxin known to inhibit receptor recycling through the inhibition of endosome acidification. Fig. 6E shows that MG132 induced Ha Ras and its effects were not additive when administered with PDGF or H₂O₂. Conversely, monensin had no effect alone or with PDGF or H₂O₂ on Ha Ras levels.

### Amplification of ROS-Ras signalling in vivo in scleroderma fibroblasts

To determine if the signalling pathway connecting ROS to Ras was relevant *in vivo*, the authors took advantage of fibroblasts derived from patients affected by systemic sclerosis. These cells produce high levels of ROS (12a) and are subjected to constitutive stimulation *in vivo* of PDGF signalling by the presence in their serum of stimulating anti-PDGF receptor antibodies. Fig. 7A shows that 3 fibroblasts lines derived from these patients contain high ROS, superoxide and H₂O₂ levels. ROS were strongly inhibited by farnesyl transferase, MEK and Ras (not shown) inhibitors (Fig. 7A). Moreover, these cells contained higher levels of Ha *Ras* protein, compared to normal controls (Fig. 7B). Ras activity was also increased relative to normal control cells (Fig. 7C). The authors have recently completed the analysis of 46 patients affected by systemic sclerosis and in all the cases the ratio Ha/Ki was higher than 2. The analysis of downstream Ras effectors (AKT and ERK1/2) indicated that only ERK1/2 was selectively activated (Fig. 7D). ROS, Ras and ERK1/2 activation were linked, because MEK inhibitors, ROS scavengers or farnesyl transferase inhibitors were able to reduce *Ras* P-ERK1/2 and ROS levels (Fig. 7A). ROS, Ha Ras and active ERK1/2 slowly decayed in cells cultured in low serum and in 1-2 days returned to the baseline (data not shown).

### Biological consequences of Ha Ras stabilization in vivo: high ROS, high ERK, DNA damage, collagen synthesis, senescence

The authors next asked if ROS-*Ras* amplification was affecting the phenotype of these cell lines. To this end, they determined: 1. activation of DNA damage checkpoints, or directly chromosomal alterations; 2. stress-induced apoptosis; 3. activation of transcription of collagen genes by ROS. Figure 8 shows that scleroderma cells contained: 1. activated ATM, assayed by phosphorylation of histone H2AX; 2. accumulation of p21 WAF (Fig. 8A), and 3. damaged chromosomes (Fig. 8B). The chromosomal aberrations were present *in vivo* before the expansion in culture and where continuously generated in culture. These alterations were amplified by ROS and resulted in negative selection of these cells (data not shown). This explains why the number of altered metaphases was significantly reduced by incubating the cells with farnesyl transferase inhibitors or ROS scavengers during the first and second day of culture (Fig. 8B). These cells were extremely sensitive to oxidative stress-induced apoptosis, which was inhibited by pre-treatment with MEK inhibitor, PD98059 (Fig. 8C). Finally, transcription of collagen genes, which was exquisitely sensitive to ROS, was greatly stimulated (Fig. 8D and 8E). All these features were inhibited by treatment of the cells with either MEK or farnesyl transferase inhibitors or ROS scavengers (Fig. 8B, 8C, 8D, 8E). To date they have replicated these data in approximately 15 independent fibroblast lines derived from systemic sclerosis patients (data not shown).

As complementary approach, they transfected normal fibroblasts with Ha or Ki *Ras* at approximately the same ratio present in scleroderma cells (3:1). The authors found that ROS production was significantly stimulated by Ha-*Ras* expression. They replicated all the features indicated in Fig. 8 (collagen induction, DNA damage, H₂O₂-induce apoptosis) by expressing Ha *Ras* in a ratio 5:1 relative to Ki *Ras* (Fig.5 Supplementary Material and ref. 15).

These data establish a link between ROS-*Ras* amplification and the complex phenotype of scleroderma fibroblasts *in vivo.* Moreover, they provide the tools for a possible diagnosis and a targeted therapy of this so far incurable illness.

### Discussion

The data indicate a novel level of regulation of Ras proteins, not known before. In established and immortal cell lines, *Ras* is solely regulated by GTP-GDP binding activity. Since widespread expression of Ha or Ki *Ras* is not tolerated during development or in adult organisms, in primary cells the levels of the proteins are maintained low (20a). In primary fibroblasts, *Ras* proteins are maintained low by proteasomal degradation (Fig.6E).

Ras induction by growth factors and ROS is not unique of primary fibroblasts but it is present also in human peripheral lymphocytes, primary neurons and primary mouse astrocytes. In these cells, H₂O₂ stimulates, both Ha and Ki *Ras*.

In primary fibroblasts the accumulation of Ras protein is triggered by PDGF and ERK1/2 (Fig.4 and 5). ROS induced by PDGF maintain ERK1/2 active (Fig.5). ROS induction of Ras is independent on PDGF stimulation and can be maintained by of ROS (Fig.6). However, in the absence of PDGF stimulation, H₂O₂ is not able to maintain high Ha Ras levels for longer periods (2 h).

As to the mechanism underlying Ha-Ras induction by PDGF and ROS, the data shown in Fig. 6E indicate that the Ha Ras protein is degraded by the 26 S proteasome and that ERK1/2 protect Ha Ras from degradation. Similarly, *c-myc* is degraded by the proteasome (21a) and is stabilized by stress via MEKK1 (22a). A schematic diagram illustrating the link between ROS and Ha-Ras protein levels is shown in Fig.9. The authors propose that ROS increase Ras protein levels and amplify PDGF signalling. The regulation of Ras protein levels protects primary cells from excessive stimulation by growth factors, which may result in apoptosis or DNA damage and oxidative stress.

### Amplification of ROS-Ras signalling in vivo

The pathway described is relevant *in vivo*, because the authors found it in cells isolated from lesions of patients affected by systemic sclerosis, an autoimmune disease, characterized by extensive fibrosis of the skin and internal organs, due to exaggerated production of collagen by fibroblasts (23a). Fibroblasts, derived from systemic sclerosis patients, contain high Ha Ras and ROS levels and constitutive activation of ERK1/2. These features are the hallmarks of the signalling pathway the authors have described above in normal fibroblasts stimulated with H₂O₂. Systemic sclerosis patients synthesize stimulating antibodies to the PDGF receptor. These antibodies stimulate fibroblasts and monocytes to produce high ROS (14a), which set off ERK1/2 and induce Ha *Ras* (Svegliati et al., submitted). Inhibition of any of the components of this loop (ERK1/2, *Ras*, ROS) downregulates the system and abolishes the biological effects of *Ras*-ROS activation, such as accelerated senescence of the cells, which characterizes the phenotype of systemic sclerosis cells (23a, 24a). These cells are 1. prone to apopotosis, 2. DNA is heavily damaged, 3. transcription of the genes induced by ROS is vigorously induced. In this framework, fibrosis is the consequence of loss of cells (apoptosis) and deposition of collagen (23a, 24a). The loop triggered initially by PDGF receptor stimulation, become relatively autonomous, since it is maintained by ROS produced by activation of NADPH oxidase by *Ras*-ERK1/2 (24). This explains why the inhibition of either ROS, or ERK1/2 or Ras converts scleroderma to normal fibroblasts. However, PDGF signaling is required for long term ROS production, since inhibition of PDGF receptor for 4-12 h reduces Ras-ROS-ERK1/2-collagen levels (data not shown).

In the presence of physiological stimuli, the regulation of *Ras* protein levels could protect primary cells from excessive or inappropriate stimuli. Coupling of ROS to *Ras* highlights the primary role of Ras proteins as sensors and controllers of redox signalling. The different turnover in primary cells and the different effects on ROS levels between Ha and Ki *Ras* (7a) suggest that the activation of these 2 isoforms signals to different cell compartments the type and the levels of ROS generated. Constitutive or mutationally activation of *Ras*-ERK1/2 signalling results in loss of this type of regulation. This may explain the opposite phenotypes on the life-span of S. cerevisiae expressing *Ras*2val19 or *Ras*2 wild type *Ras* (11a). In primary cells, senescence or growth or differentiation are likely dependent on the integrity of this circuitry.

### REFERENCES

1. Black, C.M., and Stephen, C. 1993. Systemic sclerosis (scleroderma) and related disorders. In Oxford Textbook of Rheumatology. P.J. Madison, D.A. Isemberg, P. Woo, and D.N. Glass, editors. Oxford University Press. Oxford,UK. 771-789.
2. Jimenez SA, Derk CT. Following the molecular pathway toward an understanding of the pathogenesis of systemic sclerosis Ann Inter Med 2004; 140: 37-50.
3. Stein CM, Tanner SB, Awad JA, Roberts LJ, Morrow JD. Evidence of free-radical-mediated injury (isoprostane overproduction) in scleroderma. Arthritis Rheum 1996; 39: 1146-50.
4. Bruckdorfer KR, HillaryJB, Bunce T, iVancheeswaran R, Black CM. Increased susceptibility to oxidation of low-density lipoproteins isolated from patients with systemic sclerosis. Arthritis Rheum 1995; 38: 1060- 7.
5. Casciola-Rosen L, Wigley F, Rosen A.. Scleroderma autoantigens are uniquely fragmented by metal-catalyzed oxidation reactions: implication for pathogenesis. J Exp Med 1997; 185: 71-9.
6. Sambo P, Jannino L, Candela M, et al. Monocytes of patients with systemic sclerosis (scleroderma) spontaneously release in vitro increased amounts of superoxide anion. J Invest Dermatol 1999; 112: 78-84.
7. Sambo P, Svegliati Baroni S, Padroncini P, et al. Oxidative stress in scleroderma: maintenance of scleroderma fibroblast phenotype by the constitutive upregulation of free radical generation through the NADPH oxidase complex pathway. Arthritis Rheum 2001; 44: 2653-2664.
9. Sundaresan M, Yu ZX, Ferrans VJ, Irani K, Finkel T. Requirement for generation of H2O2 for Platelet-derived growth factor signal transduction. Science 1995.: 270: 296-299.
10. Chizzolini C, Raschi E, Rezzonico R, et al. Autoantibodies to fibroblasts induce a proadhesive and pro-inflammatory fibroblast phenotype patients with systemic sclerosis Arthritis Rheum 2002: 46, 1602-13.
11. Masi AT, Rodnan GP, Medsger TA, et al. Preliminary criteria for the classification of systemic sclerosis (scleroderma). Arthritis Rheum1980; 23: 581-90.
12. LeRoy EC, Black CM, Fleischmajer R, et al. Scleroderma (Systemic sclerosis) classification, subsets and pathogenesis. J Rheumatol 1988: 12: 202-205.
13. LeRoy EC, Medsger TA. Raynaud's phenomenon: a proposal for classification. Clin Exp Rheumatol 1992; 10: 485-8.
14. Hochberg MC. 1997. Updating the American College of Rheumatology revised criteria for the classification of systemic lupus erythematosus. Arthritis Rheum 40:1725-34.
15. Arnett FC, Edworthy SM, Bloch DA, et al. The American Rheumatism Association 1987 revised criteria for the classification of rheumatoid arthritis Arthritis Rheum 1988; 31: 315-24.
16. Andrius A, Balciunaite E, Leong FL, et al. Platelet-derived growth factor plays a key role in proliferative vitreoretinopathy. Invest Ophtalmol Vis Sci 1999; 40: 283-2689.
17. Bonner JC Regulation of PDGF and its receptors in fibrotic diseases Cytok Growth Fact Rev 2004; 15: 255-273.
18. Maeda A, Hiyama k, Yamakido H, Ishioka S, Yamakido M. Increased expression of platelet-derived growth factor factor A and insulin-like growth factor -1 in BAL cells during the development of bleomycin- induced pulmonary fibrosis in mice. Chest 1996; 109: 780-786.
19. Hoyle GW, Li J, Finkelstein JB et al. Emphysematosus lesions, inflammation, and lung fibrosis in the lungs of transgenic mice overexpressing platelet-derived growth factor. Am J Pathol 1999; 154: 1763-1775.
20. Liu JK, Morris GF, Lei WH et al. Rapid activation of PDGF-A and -B expression at sites of lung injury in asbestos-exposed rats. Am J Resp Cell Mol Biol 1997; 17: 129-140.
21. Tada H, Ogushi F, Tani K et al. Increased binding and chemotactic capacities of PDGF-BB on fibroblasts in radiation pneumonitis. Radiat Res 2003; 159: 805-811.
22. Abdollahi A, Li M, Ping G et al Inhibition of platelet-derived growth factor signalling attenuates pulmonary fibrosis. J Exp Med 220; 201: 925-935.
23. Pandolfi A, Florita M, Altomare G et al Increased plasma levels of platelet-derived growth factor activity in patients with progressive systemic sclerosis. Proc Soc Exp Biol Med. 1989; 191: 1-4.
24. Ludwicka A, Ohba T, Trojanowska M et al. Elevated levels of platelet-derived growth factor and transforming growth factor-beta 1 in brochoalveolar lavage fluid from patients with scleroderma. J Rheumatol 1995; 22: 1876-1883.
25. Gay S, Jones Jr RE, Huang GQ, Gay RE. Immunohistologic demonstration of platelet-derived growth factor (PDGF) and sis-oncogene expression in scleroderma. J Invest Dermatol. 1989; 92: 301-303.
26. Klareskog L, Gustafsson R, Schynius A, Hallgren R. Increased expression of platelet-derived growth factor type B receptors in the skin of patients with systemic sclerosis. Arthritis Rheum 1990; 33: 1534-41.
27. Yamakage A, Kikuchi K, Smith EA, LeRoy EC, Trojanowska M. Selective upregulation of platelet-derived growth factor alpha receptors by transforming growth factor-beta in scleroderma fibroblasts. J Exp Med 1992; 175: 1227-34.
28. Cepeda EJ and Reveille JD. Autoantibodies in systemics sclerosis and fibrosing syndromes : clinical indications and relevance. Curr Opin Rheumatol 2004; 16: 723-732.
29. Mavalia C, Scaletti C, Romagnani P, et al. Type 2 helper T-cell predominance and high CD30 expression in systemic sclerosis. Am J Pathol 1997; 151: 1751-8.
30. Whitfield MI, Finlay DR, Murray Ji et al. Systemic and cell type-specific gene expression patterns in scleroderma skin. Proc Natl Ac Sci (USA) 2003; 100: 12319-12324.
   1a. Serrano, M., Lin, A. W., McCurrach, M. E., Beach, D. & Lowe, S. W. (1997) Cell 88, 593-602.
   2a. Chang, H., Oehrl, W., Elsner, P. & Thiele, J. J. (2003) Free Radic Res 37, 655-63.
   3a. Chen, K. C., Zhou, Y., Xing, K., Krysan, K. & Lou, M. F. (2004) Exp Eye Res 78, 1057-67.
   4a. Pani, G., Colavitti, R., Bedogni, B., Anzevino, R., Borrello, S. & Galeotti, T. (2000) J Biol Chem 275, 38891-9.
   5a. Hlavata, L., Aguilaniu, H., Pichova, A. & Nystrom, T. (2003) Embo J 22, 3337-45.
   6a. Irani, K., Xia, Y., Zweier, J. L., Sollott, S. J., Der, C. J., Fearon, E. R., Sundaresan, M., Finkel, T. & Goldschmidt-Clermont, P. J. (1997) Science 275, 1649-52.
   7a. Santillo, M., Mondola, P., Seru, R., Annella, T., Cassano, S., Ciullo, I., Tecce, M. F., lacomino, G., Damiano, S., Cuda, G., Paterno, R., Martignetti, V., Mele, E., Feliciello, A. & Awedimento, E. V. (2001) Curr Biol 11, 614-9.
   8a. Seru, R., Mondola, P., Damiano, S., Svegliati, S., Agnese, S., Awedimento, E. V. & Santillo, M. (2004) J Neurochem 91, 613-22.
   9a. Yan, Z., Chen, M., Perucho, M. & Friedman, E. (1997) J Biol Chem 272, 30928-36.
   10a. Prior, I. A. & Hancock, J. F. (2001) J Cell Sci 114, 1603-8.
   11a. Sun, J., Kale, S. P., Childress, A. M., Pinswasdi, C. & Jazwinski, S. M. (1994) J Biol Chem 269, 18638-45.
   12a. Sambo, P., Baroni, S. S., Luchetti, M., Paroncini, P., Dusi, S., Orlandini, G. & Gabrielli, A. (2001) Arthritis Rheum 44, 2653-64.
   13a. Suzukawa, K., Miura, K., Mitsushita, J., Resau, J., Hirose, K., Crystal, R. & Kamata, T. (2000) J Biol Chem 275, 13175-8.
   14a. Sambo, P., Jannino, L., Candela, M., Salvi, A., Donini, M., Dusi, S., Luchetti, M. M. & Gabrielli, A. (1999) J Invest Dermatol 112, 78-84.
   15a. Cuda, G., Paterno, R., Ceravolo, R., Candigliota, M., Perrotti, N., Perticone, F., Faniello, M. C., Schepis, F., Ruocco, A., Mele, E., Cassano, S., Bifulco, M., Santillo, M. & Awedimento, E. V. (2002) Circulation 105, 968-74.
   16a. Valletta, E. A. & Berton, G. (1987) J Immunol 138, 4366-73.
   17a. Orlandini, G., Ronda, N., Gatti, R., Gazzola, G. C. & Borghetti, A. (1999) Methods Enzymol 307, 340-50.
   18a. Feliciello, A., Gallo, A., Mele, E., Porcellini, A., Troncone, G., Garbi, C., Gottesman, M. E. & Awedimento, E. V. (2000) J Biol Chem 275, 303-11.
   19a. Kreuzer, J., Viedt, C., Brandes, R. P., Seeger, F., Rosenkranz, A. S., Sauer, H., Babich, A., Nurnberg, B., Kather, H. & Krieger-Brauer, H. I. (2003) Faseb J 17, 38-40.
   20a. Tuveson, D. A., Shaw, A. T., Willis, N. A., Silver, D. P., Jackson, E. L., Chang, S., Mercer, K. L., Grochow, R., Hock, H., Crowley, D., Hingorani, S. R., Zaks, T., King, C., Jacobetz, M. A., Wang, L., Brorison, R. T., Orkin, S. H., DePinho, R. A. & Jacks, T. (2004) Cancer Cell 5, 375-87.
   21a. Bonvini, P., Nguyen, P., Trepel, J and Neckers L.M. (1998) Oncogene, 16, 131 ± 139.
   22a. Alarcon-Vargas1, D., Tansey, W.P. and Ronai Z. (2002) Oncogene (2002) 21, 4384 ± 4391
   23a. Kahari, V. M., Vuorio, T., Nanto-Salonen, K. & Vuorio, E. (1984) Biochim Biophys Acta 781, 183-6.
   24a. Ohtsuka, T. (1998) Dermatology 196, 204-7.
   25a. Piccinini, G., Golay, J., Flora, A., Songia, S., Luchetti, M., Gabrielli, A. & Introna, M. (1999) J Invest Dermatol 112, 191-6.

## Claims

1. An *in vitro* method for detecting the presence in a body sample of auto-antibodies for the PDGF receptor **characterised by**:
a) incubating the body sample with an effective amount of a specific ligand for such auto-antibodies in conditions allowing the binding and the formation of a complex;
b) detecting the bound auto-antibodies, or the complex, if present.

2. The method of claim 1 wherein the specific ligand is the PDGF receptor or immunoreactive fragments or derivatives thereof.

3. The method of claim 2 wherein said immunoreactive fragments belong to the extracellular region of PDGFrA and/or PDGFrB.

4. The method of claim 3 wherein said immunoreactive fragments belonging to the extracellular region of PDGFrA and/or PDGFrB are comprised in the first 550 amino acids from the NH terminus of the amino acid sequence of the human PDGFrA NP008197, (SWProt P16234, SEQ ID No. 7) and/or PDGFrB NPOO26000 (SWProt P09619, SEQ ID No. 8).

5. The method of anyone of previous claims 4 for the diagnosis and prognosis of systemic sclerosis.

6. The method of claim 5 for discriminating between Raynaud phenomenon and systemic sclerosis.

7. The method of anyone of previous claims 1 to 4 for the diagnosis of the Graft-Versus-Host-Reaction (GVHR).

8. A diagnostic kit for the method according to any of previous claims comprising:
a) the PDGF receptor or immunoreactive fragments thereof for detecting auto antibodies for the PDGF receptor;
b) detecting means for bound auto-antibodies, or for the complex ligand-autoantibodies.

9. The kit of claim 8 wherein said immunoreactive fragments belong to the extracellular region of PDGFrA and/or PDGFrB.

10. The kit of claim 9 wherein said immunoreactive fragments belonging to the extracellular region of PDGFrA and/or PDGFrB are comprised in the first 550 amino acids from the NH terminus of the amino acid sequence of the human PDGFrA NP008197, (SWProt P16234, SEQ ID No. 7) and/or PDGFrB NPOO2600 (SWProt P09619, SEQ ID No. 8).

11. The PDGF receptor or immunoreactive fragments thereof for use for treating the Graft-Versus-Host-Reaction (GVHR) in patients with scleroderma-like skin lesions after allogenic bone marrow translaplantation.

12. The PDGF receptor or immunoreactive fragments thereof for use for treating the systemic sclerosis.

13. The PDGF receptor or immunoreactive fragments thereof according to claim 11 or 12 wherein said immunoreactive fragments belong to the extracellular region of PDGFrA and/or PDGFrB.

14. The PDGF receptor or immunoreactive fragments thereof according to claim 13 wherein said immunoreactive fragments belonging to the extracellular region of POGFrA and/or PDGFrB are comprised in the first 550 amino acids from the NH terminus of the amino acid sequence of the human PDGFrA NP008197, (SWProt P16234, SEQ ID No. 7) and/or PDGFrB NPOO26000 (SWProt P09619, SEQ ID No. 8).

15. A pharmaceutical composition comprising an effective amount of PDGF receptor or immunoreactive fragments thereof, and proper diluents, and /or excipients, and/or adjuvants for use in treating the Graft-Versus-Host-Reaction (GVHR) in patients with scteroderma-like skin lesions after allogenic bone marrow translaplantation or the systemic sclerosis.

16. The pharmaceutical composition of claim 15 wherein said immunoreactive fragments belong to the extracellular region of PDGFrA and/or PDGFrB.

17. The pharmaceutical composition of claim 16 wherein said immunoreactive fragments belonging to the extracellular region of PDGFrA and/or PDGFrB are comprised in the first 550 amino acids from the NH terminus of the amino acid sequence of the human PDGFrA NP008197, (SWProt P16234, SEQ ID No. 7) and/or PDGFrB NPOO26000 (SWProt P09619, SEQ ID No. 8).

## Patentansprüche

1. *In-vitro*-Verfahren zum Nachweisen des Vorhandenseins von Auto-Antikörpern gegen den PDGF-Rezeptor in einer Körperprobe, **gekennzeichnet durch**:
(a) Inkubieren der Körperprobe mit einer wirksamen Menge eines spezifischen Liganden solcher Auto-Antikörper unter Bedingungen, welche die Bindung und die Bildung eines Komplexes erlauben;
(b) Nachweisen der gebundenen Auto-Antikörper oder des Komplexes, falls vorhanden.

2. Verfahren nach Anspruch 1, wobei es sich bei dem spezifischen Liganden um den PDGF-Rezeptor oder immunreaktive Fragmente oder Derivate davon handelt.

3. Verfahren nach Anspruch 2, wobei die immunreaktiven Fragmente zu der extrazellulären Region von PDGFrA und/oder PDGFrB gehören.

4. Verfahren nach Anspruch 3, wobei die immunreaktiven Fragmente, die zu der extrazellulären Region von PDGFrA und/oder PDGFrB gehören, in den ersten 550 Aminosäuren des NH-Terminus der Aminosäuresequenz des humanen PDGFrA NP008197 (SWProt P16234, SEQ ID Nr. 7) und/oder PDGFrB NPOO2600 (SWProt P09619, SEQ ID Nr. 8) enthalten sind.

5. Verfahren nach einem der vorherigen Ansprüche 4 für die Diagnose und Prognose von systemischer Sklerose.

6. Verfahren nach Anspruch 5 zum Unterscheiden zwischen Reynaud-Phänomen und systemischer Sklerose.

7. Verfahren nach einem der vorherigen Ansprüche 1 bis 4 für die Diagnose der Graft-versus-Host-Reaktion (GVHR).

8. Diagnostisches Kit für das Verfahren nach einem der vorherigen Ansprüche, welches Folgendes aufweist:
a) den PDGF-Rezeptor oder immunreaktive Fragmente davon zum Nachweisen von Auto-Antikörpern gegen den PDGF-Rezeptor;
b) Nachweismittel für gebundene Auto-Antikörper oder für den Komplex aus Ligand und Auto-Antikörper.

9. Kit nach Anspruch 8, wobei die immunreaktiven Fragmente zu der extrazellulären Region von PDGFrA und/oder PDGFrB gehören.

10. Kit nach Anspruch 9, wobei die immunreaktiven Fragmente, die zu der extrazellulären Region von PDGFrA und/oder PDGFrB gehören, in den ersten 550 Aminosäuren des NH-Terminus der Aminosäuresequenz des humanen PDGFrA NP008197 (SWProt P16234, SEQ ID Nr. 7) und/oder PDGFrB NPOO2600 (SWProt P09619, SEQ ID Nr. 8) enthalten sind.

11. PDGF-Rezeptor oder immunreaktive Fragmente davon für die Verwendung zum Behandeln der Graft-versus-Host-Reaktion (GVHR) bei Patienten mit Sklerodermie-artigen Hautläsionen nach allogener Knochenmarktransplantation.

12. PDGF-Rezeptor oder immunreaktive Fragmente davon für die Verwendung zum Behandeln der systemischen Sklerose.

13. PDGF-Rezeptor oder immunreaktive Fragmente davon nach Anspruch 11 oder 12, wobei die immunreaktiven Fragmente zu der extrazellulären Region von PDGFrA und/oder PDGFrB gehören.

14. PDGF-Rezeptor oder immunreaktive Fragmente davon nach Anspruch 13, wobei die immunreaktiven Fragmente, die zu der extrazellulären Region von PDGFrA und/oder PDGFrB gehören, in den ersten 550 Aminosäuren des NH-Terminus der Aminosäuresequenz des humanen PDGFrA NP008197 (SWProt P16234, SEQ ID Nr. 7) und/oder PDGFrB NPOO2600 (SWProt P09619, SEQ ID Nr. 8) enthalten sind.

15. Pharmazeutische Zusammensetzung, welche eine wirksame Menge an PDGF-Rezeptor oder immunreaktiven Fragmenten davon und geeignete Verdünnungsmittel und/oder Exzipienten und/oder Adjuvanzien aufweist, für die Verwendung zum Behandeln der Graft-versus-Host-Reaktion (GVHR) bei Patienten mit Sklerodermie-artigen Hautläsionen nach allogener Knochenmarktransplantation oder der systemischen Sklerose.

16. Pharmazeutische Zusammensetzung nach Anspruch 15, wobei die immunreaktiven Fragmente zu der extrazellulären Region von PDGFrA und/oder PDGFrB gehören.

17. Pharmazeutische Zusammensetzung nach Anspruch 16, wobei die immunreaktiven Fragmente, die zu der extrazellulären Region von PDGFrA und/oder PDGFrB gehören, in den ersten 550 Aminosäuren des NH-Terminus der Aminosäuresequenz des humanen PDGFrA NP008197 (SWProt P16234, SEQ ID Nr. 7) und/oder PDGFrB NPOO2600 (SWProt P09619, SEQ ID Nr. 8) enthalten sind.

## Revendications

1. Procédé in vitro de détection de la présence dans un échantillon corporel d'auto-anticorps du récepteur PDGF **caractérisé par** :
a) l'incubation de l'échantillon corporel avec une quantité efficace d'un ligand spécifique pour ces auto-anticorps dans des conditions permettant la liaison et la formation d'un complexe ;
b) la détection des auto-anticorps liés, ou du complexe, s'ils sont présents.

2. Procédé selon la revendication 1, le ligand spécifique étant le récepteur PDGF ou des fragments immunoréactifs ou des dérivés de celui-ci.

3. Procédé selon la revendication 2, lesdits fragments immunoréactifs appartenant à la région extracellulaire de PDGFrA et/ou de PDGFrB.

4. Procédé selon la revendication 3, lesdits fragments immunoréactifs qui appartiennent à la région extracellulaire de PDGFrA et/ou de PDGFrB étant compris dans les 550 premiers acides aminés à partir de l'extrémité NH terminale de la séquence d'acides aminés du PDGFrA NP008197, (SWProt P16234, SEQ ID NO : 7) et/ou du PDGFrB NP0026000 (SWProt P09619, SEQ ID NO : 8) humains.

5. Procédé selon l'une quelconque des revendications précédentes, destiné au diagnostic et au pronostic de la sclérodermie.

6. Procédé selon la revendication 5, destiné à faire la différence entre le syndrome de Raynaud et la sclérodermie.

7. Procédé selon l'une quelconque des revendications précédentes 1 à 4, destiné au diagnostic de la maladie du greffon contre l'hôte (GVHR).

8. Kit de diagnostic pour le procédé selon l'une quelconque des revendications précédentes, qui comprend :
a) le récepteur PDGF ou des fragments immunoréactifs de celui-ci pour la détection d'auto-anticorps du récepteur PDGF ;
b) un moyen de détection des auto-anticorps liés, ou du complexe ligand-anticorps.

9. Kit selon la revendication 8, les fragments immunoréactifs appartenant à la région extracellulaire de PDGFrA et/ou de PDGFrB.

10. Kit selon la revendication 9, lesdits fragments immunoréactifs qui appartiennent à la région extracellulaire de PDGFrA et/ou de PDGFrB étant compris dans les 550 premiers acides aminés à partir de l'extrémité NH terminale de la séquence d'acides aminés du PDGFrA NP008197, (SWProt P16234, SEQ ID NO : 7) et/ou du PDGFrB NP0026000 (SWProt P09619, SEQ ID NO : 8) humains.

11. Récepteur PDGF ou fragments immunoréactifs de celui-ci destinés à être utilisés pour le traitement de la maladie du greffon contre l'hôte (GVHR) chez des patients présentant des lésions cutanées de type sclérodermie après une transplantation de moelle osseuse allogénique.

12. Récepteur PDGF ou fragments immunoréactifs de celui-ci destinés au traitement de la sclérodermie.

13. Récepteur PDGF ou fragments immunoréactifs de celui-ci selon la revendication 11 ou 12, lesdits fragments immunoréactifs appartenant à la région extracellulaire de PDGFrA et/ou de PDGFrB.

14. Récepteur PDGF ou fragments immunoréactifs de celui-ci selon la revendication 13, lesdits fragments immunoréactifs qui appartiennent à la région extracellulaire de PDGFrA et/ou de PDGFrB étant compris dans les 550 premiers acides aminés à partir de l'extrémité NH terminale de la séquence d'acides aminés du PDGFrA NP008197, (SWProt P16234, SEQ ID NO : 7) et/ou du PDGFrB NP0026000 (SWProt P09619, SEQ ID NO : 8) humains.

15. Composition pharmaceutique comprenant une quantité efficace de récepteur PDGF ou de fragments immunoréactifs de celui-ci, et des diluants et/ou excipients et/ou adjuvants adaptés, destinée à être utilisée dans le traitement de la maladie du greffon contre l'hôte (GVHR) chez des patients présentant des lésions cutanées de type sclérodermie après une transplantation de moelle osseuse allogénique ou de la sclérodermie.

16. Composition pharmaceutique selon la revendication 15, les fragments immunoréactifs appartenant à la région extracellulaire de PDGFrA et/ou de PDGFrB.

17. Composition pharmaceutique selon la revendication 16, lesdits fragments immunoréactifs qui appartiennent à la région extracellulaire de PDGFrA et/ou de PDGFrB étant compris dans les 550 premiers acides aminés à partir de l'extrémité NH terminale de la séquence d'acides aminés du PDGFrA NP008197, (SWProt P16234, SEQ ID NO : 7) et/ou du PDGFrB NP0026000 (SWProt P09619, SEQ ID NO : 8) humains.


### SEQUENCE LISTING

<110> Avvedimento et al.
   Avvedimento, Vittorio Enrico
   santillo, Mariarosaria
   Funaro, Ada
   Lucchetti, Michele
   Svegliati Baroni, Silvia
   Gabrielli, Armando
<120> STIMULATORY AUTO-ANTIBODIES TO THE PDGF RECEPTOR AS PATHOLOGY
   MARKERS AND THERAPEUTIC TARGET
<130> PCT 94780
<150> US 60/703,377
   <151> 2005-07-28
<160> 8
<170> PatentIn version 3.3
<210> 1
   <211> 20
   <212> DNA
   <213> Homo sapiens
<400> 1
   acatctcttt gctgcccaat 20
<210> 2
   <211> 20
   <212> DNA
   <213> Homo sapiens
<400> 2
   gagcgagact ctgacaccaa 20
<210> 3
   <211> 20
   <212> DNA
   <213> Homo sapiens
<400> 3
   tcgacacagc aggtcaagag 20
<210> 4
   <211> 20
   <212> DNA
   <213> Homo sapiens
<400> 4
   aggcatcatc aacaccctgt 20
<210> 5
   <211> 20
   <212> DNA
   <213> Homo sapiens
<400> 5
   ccagctgatc cagaaccatt 20
<210> 6
   <211> 20
   <212> DNA
   <213> Homo sapiens
<400> 6 20
   aggtctcgat gtaggggatg 20
<210> 7
   <211> 1089
   <212> PRT
   <213> Homo sapiens
<400> 7
<210> 8
   <211> 1106
   <212> PRT
   <213> Homo sapiens
<400> 8